# EUROPÄISCHE PATENTSCHRIFT

(11) **EP 0 439 785 B1**
(45) Veröffentlichungstag und Bekanntmachung des Hinweises auf die Patenterteilung: **27.09.1995**
(21) Anmeldenummer: 90124678.5
(22) Anmeldetag: 19.12.1990
(51) Int. Cl.: C07D 403/04, C07D 403/14, A01N 43/54, C07D 401/14

(54) **Substituierte 2-[6-(Pyrimidinyl)-indol-1-yl]-acrylsäureester**
Substituted 2-[6-(pyrimidinyl)-indol-1-yl]-acrylic acid ester
Ester d'acide 2-[6-(pyrimidinyl)-indol-1-yl]-acrylique substitué

(30) Priorität: 28.01.1990 DE 4002466
(43) Veröffentlichungstag der Anmeldung: 07.08.1991
(73) Patentinhaber: BAYER AG, 51368 Leverkusen (DE)
(72) Erfinder: Seitz, Thomas, Dr., W-4019 Monheim (DE); Klausener, Alexander, Dr., W-4150 Krefeld 1 (DE); Berg, Dieter, Dr., W-5600 Wuppertal 1 (DE); Wachendorff-Neumann, Ulrike, Dr., W-4019 Monheim (DE); Erdelen, Christoph, Dr., W-5653 Leichlingen 3 (DE); Hänssler, Gerd, Dr., W-5090 Leverkusen 3 (DE); Brandes, Wilhelm, Dr., W-5653 Leichlingen 1 (DE); Dutzmann, Stefan, Dr., W-4000 Düsseldorf 13 (DE)

(56) Entgegenhaltungen:
- EP-A- 0 206 523
- EP-A- 0 329 011

## Beschreibung

Die Erfindung betrifft neue substituierte 2-[6-(Pyrimidinyl)-indol-1-yl]-acrylsäureester, neue Zwischenprodukte sowie mehrere Verfahren zu ihrer Herstellung und ihre Verwendung als Schädlingsbekämpfungsmittel.

Es ist bekannt, daß bestimmte substituierte Acrylsäureester, wie beispielsweise die Verbindung 3-Methoxy-2-(2-methylphenyl)acrylsäuremethylester fungizide Eigenschaften besitzen (vgl. z.B. EP-A 178826, EP-A- 206523).

Weiterhin ist bekannt, daß bestimmte in 2-Stellung durch einen 1-Indolylrest substituierte Alkoxyacrylsäureester, wie beispielsweise die Verbindung 3-Methoxy-2-(indol-1-yl)-acrylsäuremethylester, fungizid wirksam sind (vgl. EP-A 274825, EP-A- 329011).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue substituierte 2-[6-(Pyrimidinyl)-indol-1-yl]-acrylsäureester der allgemeinen Formel (I) gefunden, 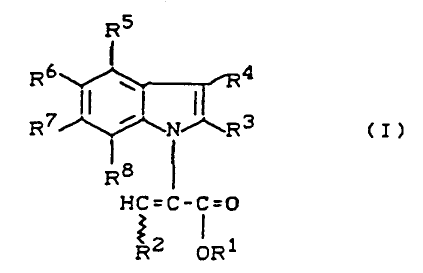
in welcher
- R¹: für geradkettiges oder verzweigtes Alkyl mit 1 bis 6 Kohlenstoffatomen oder für Benzyl steht,
- R²: für Dialkylamino mit jeweils 1 bis 6 Kohlenstoffatomen in den einzelnen geradkettigen oder verzweigten Alkylteilen, jeweils geradkettiges oder verzweigtes Alkoxy oder Alkylthio mit 1 bis 6 Kohlenstoffatomen oder für jeweils gegebenenfalls einfach oder mehrfach, gleich oder verschieden substituiertes Benzyloxy oder Benzylthio steht, wobei als Phenylsubstituenten infrage kommen:
Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen oder Cycloalkyl mit 3 bis 7 Kohlenstoffatomen,
- R³ und R⁴: jeweils unabhängig voneinander für Wasserstoff, Cyano, Fluor, Chlor, Brom oder geradkettiges oder verzweigtes Alkyl mit 1 bis 6 Kohlenstoffatomen stehen,
- R⁵, R⁶ und R⁸: unabhängig voneinander jeweils für Wasserstoff, Fluor, Chlor, Brom, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Cycloalkyl mit 3 bis 7 Kohlenstoffatomen, jeweils unsubstituiertes oder im Arylteil einfach bis dreifach, gleich oder verschieden durch Fluor, Chlor, Brom, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Phenyl, Benzyl, Phenoxy, Phenylthio, Benzyloxy oder Benzylthio oder jeweils gegebenenfalls im Heteroarylteil einfach oder zweifach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Pyridyl, Thienyl, Furyl, Pyridazinyl, Pyrazinyl, Thiazolyl, Pyridylmethyl, Thienylmethyl, Furylmethyl, Pyridyloxy, Thienyloxy, Thiazolyloxy, Pyridylmethyloxy, Thienylmethyloxy, Thienylthio, Furylthio, Pyridylmethylthio oder Thienylmethylthio stehen, oder
- R⁵ und R⁶: gemeinsam für Alkandiyl mit 3 bis 5 Kohlenstoffatomen oder für Alkylidendioxy mit 1 bis 3 Kohlenstoffatomen stehen und
- R⁷: für eine der folgenden Gruppierungen steht 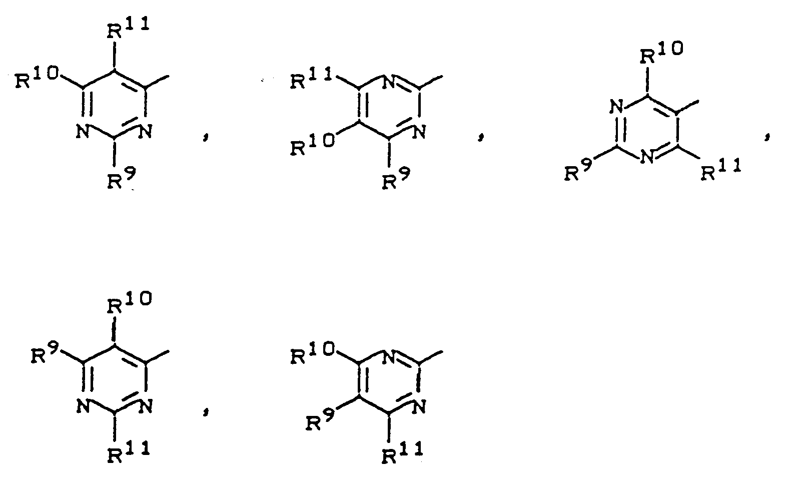 wobei
- R⁹, R¹⁰ und R¹¹: jeweils unabhängig voneinander für Wasserstoff, Fluor, Chlor, Brom, Cyano, jeweils geradkettiges oder verzweigtes Alkyl, Alkoxy, Alkylthio oder Halogenalkyl mit 1 bis 6 Kohlenstoffatomen und gegebenenfalls 1 bis 13 gleichen oder verschiedenen Halogenatomen steht, für Cycloalkyl mit 3 bis 7 Kohlenstoffatomen, für geradkettiges oder verzweigtes Alkoxycarbonyl mit 1 bis 6 Kohlenstoffatomen, für jeweils in den einzelnen Alkylteilen geradkettiges oder verzweigtes, gegebenenfalls gleich oder verschieden substituiertes Dialkylaminocarbonyl, Alkylaminocarbonyl, Dialkylamino oder Alkylamino mit jeweils 1 bis 4 Kohlenstoffatomen oder für jeweils unsubstituiertes oder einfach bis dreifach, gleich oder verschieden substituiertes Phenyl, Benzyl, Phenyloxy, Phenylthio, Benzyloxy oder Benzylthio steht oder für unsubstituiertes oder einfach oder zweifach, gleich oder verschieden substituiertes Pyrrolyl, Thienyl, Furyl, Thiazolyl, Isothiazolyl, Oxazolyl, Isoxazolyl, Pyrazolyl, Imidazolyl, Pyridyl, Pyrimidyl, Pyridazinyl, Pyrazinyl, Triazinyl, Pyrrolyloxy, Thienyloxy, Furyloxy, Thiazolyloxy, Isothiazolyloxy, Oxazolyloxy, Isoxazolyloxy, Pyrazolyloxy, Imidazolyloxy, Pyridyloxy, Pyrimidyloxy, Pyridazinyloxy, Pyrazinyloxy, Pyrrolylthio, Thienylthio, Furylthio, Thiazolylthio, Isothiazolylthio, Oxazolylthio, Isoxazolylthio, Pyrazolylthio, Imidazolylthio, Pyridylthio, Pyrimidylthio, Pyridazinylthio oder Pyrazinylthio steht, wobei als Substituenten jeweils infrage kommen: Halogen, jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und jeweils gegebenenfalls 1 bis 9 gleichen oder verschiedenen Halogenatomen, Alkylcarbonylamino mit 1 bis 4 Kohlenstoffatomen im geradkettigen oder verzweigten Alkylteil, Dialkylamino oder Dialkylaminocarbonyl mit jeweils 1 bis 4 Kohlenstoffatomen in den jeweiligen geradkettigen oder verzweigten Alkylteilen sowie jeweils gegebenenfalls substituiertes Phenyl oder Benzyl, oder
zwei benachbarte Substituenten R⁹ und R¹⁰, bzw. R¹⁰ und R¹¹ oder R⁹ und R¹¹ gemeinsam für Alkandiyl mit 3 oder 4 Kohlenstoffatomen stehen.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemaß beansprucht.

Weiterhin wurde gefunden, daß man die neuen substituierten 2-[6-(Pyrimidinyl)-indol-1-yl]-acrylsäureester der allgemeinen Formel (I) 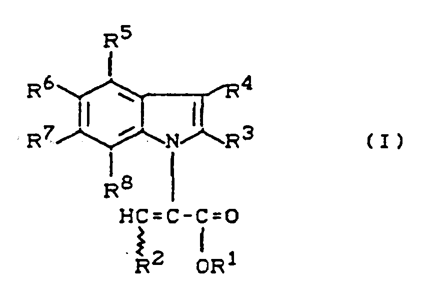
in welcher
- R¹, R², R³, R⁴, R⁵, R⁶, R⁷ und R⁸: die oben angegebene Bedeutung haben
nach einem der im folgenden beschriebenen Verfahren erhält:
a) Man erhält substituierte 2-[6-(Pyrimidinyl)-indol-1-yl]-acrylsäureester der allgemeinen Formel (Ia) 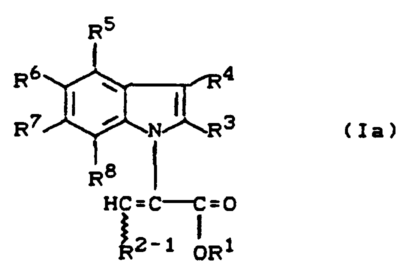 in welcher
   - R¹, R³, R⁴, R⁵, R⁶, R⁷ und R⁸: die oben angegebene Bedeutung haben und
   - R²⁻¹: für Alkoxy oder unsubstituiertes oder substituiertes Aralkyloxy steht,
   wenn man Hydroxyacrylsäureester oder deren Alkalimetallsalze der Formel (II) 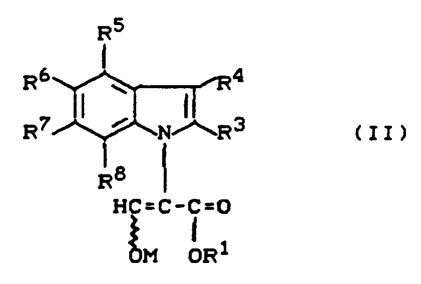 in welcher
   - M: für Wasserstoff oder für ein Alkalimetallkation steht und
   - R¹, R³, R⁴, R⁵, R⁶, R⁷ und R⁸: die oben angegebene Bedeutung haben,
   mit Alkylierungsmitteln der Formel (III)

   R¹²-E¹ (III)

   in welcher
   - R¹²: für Alkyl oder unsubstituiertes oder substituiertes Aralkyl steht und
   - E¹: für eine elektronenanziehende Abgangsgruppe steht,
   gegebenenfalls in Gegenwart eines Verdünnungsmittels und gegebenenfalls in Gegenwart eines Realtionshilfsmittels umsetzt;
b) man erhält substituierte 2-[6-(Pyrimidinyl)-indol-1-yl]-acrylsäureester der allgemeinen Formel (Ib) 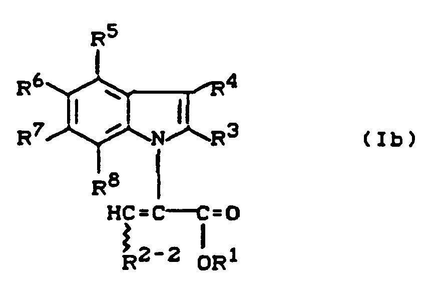 in welcher
   - R¹, R³, R⁴, R⁵, R⁶, R⁷ und R⁸: die oben angegebene Bedeutung haben und
   - R²⁻²: für Dialkylamino steht,
   wenn man substituierte Essigsäureester der Formel (IV) 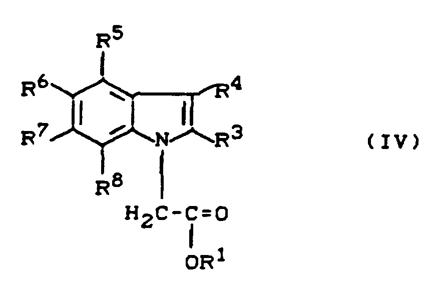 in welcher
   - R¹, R³, R⁴, R⁵, R⁶, R⁷ und R⁸: die oben angegebene Bedeutung haben,
   mit Formamiden der Formel (Va) 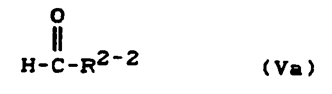 in welcher
   - R²⁻²: die oben angegebene Bedeutung hat,
   oder mit Formamid-Derivaten der Formel (Vb) 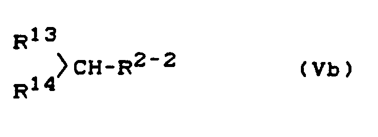 in welcher
   - R¹³ und R¹⁴: unabhängig voneinander für Alkoxy oder Dialkylamino stehen und
   - R²⁻²: die oben angegebene Bedeutung hat,
   gegebenenfalls in Gegenwart eines Verdünnungsmittels umsetzt;
c) man erhält substituierte 2-[6-(Pyrimidinyl)-indol-1-yl]-acrylsäureester der Formel (Ic) 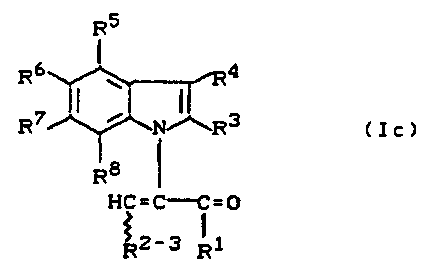 in welcher
   - R¹, R³, R⁴, R⁵, R⁶, R⁷ und R⁸: die oben angegebene Bedeutung haben und
   - R²⁻³: für Alkylthio oder unsubstituiertes oder substituiertes Aralkylthio steht,
   wenn man Ketocarbonsäurederivate der Formel (VI) 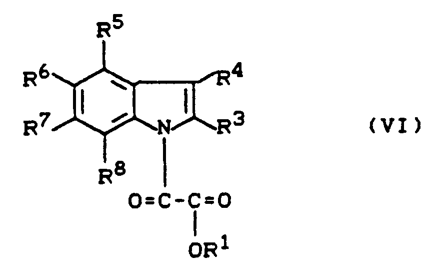 in welcher
   - R¹, R³, R⁴, R⁵, R⁶, R⁷ und R⁸: die oben angegebene Bedeutung haben,
   mit metallorganischen Verbindungen der Formel (VII) 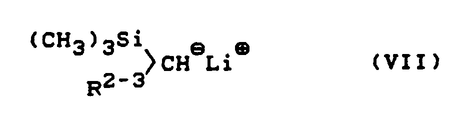 in welcher
   - R²⁻³: die oben angegebene Bedeutung hat,
   gegebenenfalls in Gegenwart eines Verdünnungsmittels umsetzt;
d) man erhält substituierte 2-[6-(Pyrimidinyl)-indol-1-yl]-acrylsäureester der Formel (Ic) weiterhin,
   wenn man substituierte Acrylsäureester der Formel (VIII) 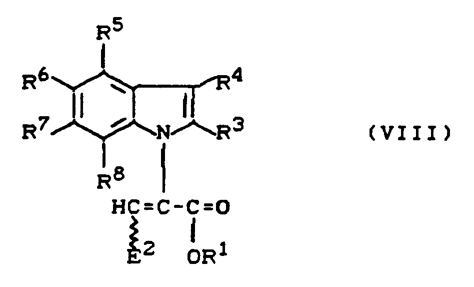 in welcher
   - R¹, R³, R⁴, R⁵, R⁶, R⁷ und R⁸: die oben angegebene Bedeutung haben und
   - E²: für eine elektronenanziehende Abgangsgruppe steht,
   mit Thiolen der Formel (IX)

   R²⁻³-H (IX)

   in welcher
   - R²⁻³: die oben angegebene Bedeutung hat,
   gegebenenfalls in Gegenwart eines Verdünnungsmittels und gegebenenfalls in Gegenwart eines Reaktionshilfsmittels umsetzt.

Schließlich wurde gefunden, daß die neuen substituierten 2-[6-(Pyrimidinyl)-indol-1-yl]-acrylsäureester der allgemeinen Formel (I) eine gute Wirkung gegen landwirtschaftliche Schädlinge besitzen.

Überraschenderweise zeigen die erfindungsgemäßen substituierten 2-[6-(Pyrimidinyl)-indol-1-yl]-acrylsäureester der allgemeinen Formel (I) eine insektizide Wirkung sowie eine erheblich bessere fungizide Wirksamkeit als die aus dem Stand der Technik bekannten Acrylsäureester, wie beispielsweise die Verbindung 3-Methoxy-2-(2-methylphenyl)-acrylsäuremethylester, welche strukturell und wirkungsmäßig naheliegende Verbindungen sind.

Bevorzugte Substituenten bzw. Bereiche der in den oben und nachstehend erwähnten Formeln aufgeführten Reste werden im folgenden erläutert:
Für Alkyl in den Definitionen von R¹, R³, R⁴, R⁵, R⁶, R⁸, R⁹, R¹⁰ und R¹¹ in den allgemeinen Formeln, steht geradkettiges oder verzweigtes Alkyl mit vorzugsweise 1 bis 8, besonders bevorzugt 1 bis 6 und insbesondere 1 bis 4, Kohlenstoffatomen. Beispielhaft seien Methyl, Ethyl, n-Propyl, i-Propyl, n-Butyl, sec-Butyl, i-Butyl, t-Butyl, n-Pentyl, i-Pentyl, t-Pentyl und n-Hexyl genannt.

Dialkylamino in der Definition von R² oder in Zusammensetzungen wie Dialkylaminocarbonyl in der Definition von R⁹, R¹⁰ und R¹¹ steht für eine Aminogruppe mit 2 Alkylgruppen, welche jeweils geradkettig oder verzweigt, gleich oder verschieden sein können und vorzugsweise jeweils 1 bis 6, insbesondere 1 bis 4 Kohlenstoffatome enthalten, wobei Methyl, Ethyl, n- und i-Propyl genannt seien.

Beispielhaft seien Dimethylamino, Diethylamino, Di-n-propylamino und Di-i-propylamino aufgeführt.

Unter dem Begriff unsubstituiertes oder substituiertes Aryl in der Definition von R⁵, R⁶, R⁸, R⁹, R¹⁰ und R¹¹ in den allgemeinen Formeln ist Aryl mit vorzugsweise 6 bis 10 Kohlenstoffatomen im Arylteil zu verstehen. Beispielhaft und vorzugsweise seien unsubstituiertes oder substituiertes Phenyl oder Naphthyl, insbesondere Phenyl genannt.

Unsubstituiertes oder substituiertes Aralkyl in den Definitionen von R¹, R⁵, R⁶, R⁸, R⁹, R¹⁰ und R¹¹ enthält vorzugsweise 1 bis 6, insbesondere 1 bis 4 Kohlenstoffatome im geradkettigen oder verzweigten Alkylteil und vorzugsweise Phenyl als Arylteil. Als Aralkylgruppen seien beispielhaft und vorzugsweise Benzyl und Phenethyl genannt.

Heteroaryl in der Definition von R⁵, R⁶, R⁸, R⁹, R¹⁰ und R¹¹ steht im allgemeinen für einen 5- bis 6-gliedrigen Ring, der ein bis 4, bevorzugt 1 bis 3 gleiche oder verschiedene Heteroatome, enthält. Als Heteroatome seien vorzugsweise Sauerstoff, Schwefel und Stickstoff genannt; beispielhaft und vorzugsweise seien genannt: Pyrimidinyl, Pyrrolyl, Isothiazolyl, Oxazolyl, Pyridyl, Thienyl, Furyl, Pyridazinyl, Pyrazinyl, Isoxazolyl, Thiazolyl und Pyrazolyl.

Unter dem Begriff Alkoxy in der Definition von R², R⁵, R⁶, R⁸, R⁹, R¹⁰ und R¹¹ in den allgemeinen Formeln ist geradkettiges oder verzweigtes Alkoxy mit vorzugsweise 1 bis 6, insbesondere 1 bis 4 Kohlenstoffatomen zu verstehen. Beispielhaft seien Methoxy, Ethoxy, Propoxy, Butoxy sowie ihre Isomeren, i-Propoxy, i-, s- und t-Butoxy genannt.

Halogen steht in den Definitionen R³, R⁴, R⁵, R⁶, R⁸, R⁹, R¹⁰ und R¹¹ für Fluor, Chlor, Brom und Iod, bevorzugt für Fluor, Chlor und Brom, besonders bevorzugt für Fluor und Chlor.

Alkyl steht in Zusammensetzungen wie Alkoximinoalkyl in den Definitionen R⁵, R⁶ und R⁸ für geradkettiges oder verzweigtes Alkyl, bevorzugt mit 1 bis 6, besonders bevorzugt mit 1 bis 4 Kohlenstoffatomen, ganz besonders bevorzugt sind Methyl, Ethyl und t-Butyl. Die beispielhafte Aufzählung entspricht der weiter oben gegebenen.

Alkylthio steht in den Definitionen R², R⁵, R⁶, R⁸, R⁹, R¹⁰ und R¹¹ für geradkettiges oder verzweigtes Alkylthio mit vorzugsweise 1 bis 6 Kohlenstoffatomen, beispielsweise sind darunter die folgenden Gruppen zu verstehen: Methylthio-, Ethylyhio-, Propylthio-, Butylthio-, Pentylthio sowie ihre Isomeren, wie z.B. i-Propylthio, i-, s- und t-Butylthio, 1-Methyl-butylthio, 2-Methyl-butylthio- und 3-Methyl-butylthio. Bevorzugte Alkylthioreste enthalten 1 bis 4 Kohlenstoffatome. Besonders bevorzugt sind Methylthio, Ethylthio, n-, i-, s-Propylthio und n-, i-, s- und t-Butylthio.

Halogenalkyl und Halogenalkoxy stehen in den Definitionen von R⁵, R⁶, R⁸, R⁹, R¹⁰ und R¹¹ für geradkettiges oder verzweigtes Halogenalkyl oder Halogenalkoxy mit je 1 bis 4 Kohlenstoffatomen, besonders bevorzugt mit 1 oder 2 Kohlenstoffatomen und jeweils 1 bis 9, vorzugsweise 1 bis 5, gleichen oder verschiedenen Halogenatomen wie unter Halogen definiert; beispielhaft seien genannt: Fluormethyl, Chlormethyl, Brommethyl, Fluorethyl, Chlorethyl, Brommethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlordifluor-methyl, Trifluorchlorethyl, Chlorbutyl, Fluorbutyl, Fluormethoxy, Chlormethoxy, Brommethoxy, Fluorethoxy, Chlorethoxy, Bromethoxy, Fluorpropoxy, Chlorpropoxy, Brompropoxy, Fluorbutoxy, Chlorbutoxy, Fluor-i-propoxy, Chlor-i-propoxy, Difluormethoxy, Trifluormethoxy, Dichlormethoxy, Trichlormethoxy, Difluorethoxy, Trifluorethoxy, Tetrafluorethoxy, Trichlorethoxy, Chlordifluormethoxy und Trifluorchlorethoxy.

Halogenalkylthio steht in den Definitionen von R⁵, R⁶ und R⁸ für geradkettiges oder verzweigtes Halogenalkylthio mit je 1 bis 4 Kohlenstoffatomen, besonders bevorzugt mit 1 oder 2 Kohlenstoffatomen und jeweils 1 bis 9, vorzugsweise 1 bis 5, gleichen oder verschiedenen Halogenatomen wie unter Halogen definiert; beispielhaft seien genannt: Fluormethylthio, Chlormethylthio, Brommethylthio, Fluorethylthio, Chlorethylthio, Brommethylthio, Fluorpropylthio, Chlorpropylthio, Brompropylthio, Fluorbutylthio, Chlorbutylthio, Brombutylthio, Fluor-i-propylthio, Chlor-i-propylthio, Difluormethylthio, Trifluormethylthio, Dichlormethylthio, Trichlormethylthio, Difluorethylthio, Trifluorethylthio, Tetrafluorethylthio, Trichlorethylthio, Chlordifluormethylthio und Trifluorchlorethylthio.

Alkoxycarbonyl steht in den Definitionen R⁵, R⁶, R⁸, R⁹, R¹⁰ und R¹¹ für geradkettiges oder verzweigtes Alkoxycarbonyl mit 1 bis 4, vorzugsweise 1 oder 2 Kohlenstoffatomen im Alkoxyrest; beispielhaft seien genannt: Methoxycarbonyl, Ethoxycarbonyl, n-Propoxycarbonyl, i-Propoxycarbonyl, n-, i-, s- und t-Butoxycarbonyl.

Cycloalkyl steht in den Definitionen R⁵, R⁶ und R⁸ für Cycloalkyl mit vorzugsweise 3 bis 7, insbesondere 3, 5 oder 6 Kohlenstoffatomen, Beispielhaft seien unsubstituiertes oder substituiertes Cyclopropyl, Cyclobutyl, Cyclopentyl, Cyclohexyl und Cycloheptyl genannt.

Unsubstituiertes oder substituiertes Aryloxy und Arylthio stehen in den Definitionen von R⁵, R⁶, R⁸ und R⁹ in den allgemeinen Formeln für Aryl mit vorzugsweise 6 bis 10 Kohlenstoffatomen im Arylteil. Beispielhaft seien unsubstituiertes oder substituiertes Phenyl oder Naphthyl, Phenoxy oder Phenylthio, insbesondere Phenyl genannt.

Unsubstituiertes oder substituiertes Aralkyloxy oder Aralkylthio enthalten in den Definitionen R², R⁵, R⁶, R⁸, R⁹, R¹⁰ und R¹¹ vorzugsweise 1 bis 6 Kohlenstoffatome im geradkettigen oder verzweigten Alkylteil und vorzugsweise Phenyl als Arylteil. Als Aralkylgruppen seien beispielhaft Benzyl, Phenethyl, Benzyloxy und Benzylthio genannt.

Hetarylalkyl, Hetaryloxy und Hetarylthio in der Definition von R⁵, R⁶, R⁸, R⁹, R¹⁰ und R¹¹ stehen im allgemeinen für einen 5- bis 6-gliedrigen Ring, der ein oder mehrere Heteroatome, bevorzugt 1 bis 3 gleiche oder verschiedene Heteroatome, enthält. Als Heteroatome seien vorzugsweise Sauerstoff, Schwefel und Stickstoff genannt; beispielhaft seien genannt: Pyridyl, Thienyl, Furyl, Pyridazinyl, Pyrazinyl, Isoxazolyl, Thiazolyl, Pyridylmethyl, Thienylmethyl, Furylmethyl, Pyridyloxy, Thienyloxy, Furyloxy, Pyridazinyloxy, Pyrazinyloxy, Isoxazolyloxy, Thiazolyloxy, Pyridylmethyloxy, Thienylmethyloxy, Furylmethyloxy, Pyridylthio, Thienylthio, Furylthio, Pyridazinylthio, Pyrazinylthio, Isoxazolylthio, Thiazolylthio, Pyridylmethylthio, Thienylmethylthio und Furylmethylthio.

Die Substituenten für die Arylreste als solche oder in Zusammensetzungen wie Arylalkyl, Aryloxy, Arylthio, Aralkyloxy und für die heterocyclischen Ringe wie Hetarylalkyl und Hetaryl haben die im folgenden angegebenen Bedeutungen.

Halogen steht im allgemeinen als Substituent für Fluor, Chlor, Brom und Iod, bevorzugt für Fluor, Chlor und Brom, besonders bevorzugt für Fluor und Chlor.

Alkyl steht im allgemeinen als Substituent oder in Zusammensetzungen wie Alkoximinoalkyl für geradkettiges oder verzweigtes Alkyl, bevorzugt mit 1 bis 6, besonders bevorzugt mit 1 bis 4 Kohlenstoffatomen, ganz besonders bevorzugt sind Methyl, Ethyl, i-Propyl und t-Butyl. Die beispielhafte Aufzählung entspricht der weiter oben gegebenen.

Alkoxy steht im allgemeinen als Substituent oder in Zusammensetzungen wie Alkoximinoalkyl für geradkettiges oder verzweigtes Alkoxy mit 1 bis 6, besonders bevorzugt 1 bis 3 Kohlenstoffatomen je Alkylrest; beispielhaft und vorzugsweise seien genannt: Methoxy, Ethoxy, n-und i-Propoxy, n-, i-, s- und t-Butoxy, n-Hexoxy und i-Hexoxy.

Alkylthio steht im allgemeinen als Substituent in den Resten für geradkettiges oder verzweigtes Alkylthio mit vorzugsweise 1 bis 6 Kohlenstoffatomen, beispielsweise sind darunter die folgenden Gruppen zu verstehen: Methylthio-, Ethylthio-, Propylthio-, Butylthio-, Pentylthio sowie ihre Isomeren, wie z.B. i-Propylthio, i-, s- und t-Butylthio, 1-Methyl-butylthio, 2-Methyl-butylthio- und 3-Methyl-butylthio. Bevorzugte Alkylthioreste enthalten 1 bis 4 Kohlenstoffatome. Besonders bevorzugt sind Methylthio, Ethylthio, n-, i-, s-Propylthio und n-, i-, s- und t-Butylthio.

Halogenalkyl und Halogenalkoxy stehen im allgemeinen als Substituenten in den Resten für geradkettiges oder verzweigtes Halogenalkyl oder Halogenalkoxy mit je 1 bis 4 Kohlenstoffatomen, besonders bevorzugt mit 1 oder 2 Kohlenstoffatomen und jeweils 1 bis 9, vorzugsweise 1 bis 5, gleichen oder verschiedenen Halogenatomen wie unter Halogen definiert; beispielhaft seien genannt: Fluormethyl, Chlormethyl, Brommethyl, Fluorethyl, Chlorethyl, Brommethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlor-difluor-methyl, Trifluorchlorethyl, Chlorbutyl, Fluorbutyl, Fluormethoxy, Chlormethoxy, Brommethoxy, Fluorethoxy, Chlorethoxy, Bromethoxy, Fluorpropoxy, Chlorpropoxy, Brompropoxy, Fluorbutoxy, Chlorbutoxy, Fluor-i-propoxy, Chlor-i-propoxy, Difluormethoxy, Trifluormethoxy, Dichlormethoxy, Trichlormethoxy, Difluorethoxy, Trifluorethoxy, Tetrafluorethoxy, Trichlorethoxy, Chlordifluormethoxy und Trifluorchlorethoxy.

Halogenalkylthio steht im allgemeinen als Substituent in den Resten für geradkettiges oder verzweigtes Halogenalkylthio mit je 1 bis 4 Kohlenstoffatomen, besonders bevorzugt mit 1 oder 2 Kohlenstoffatomen und jeweils 1 bis 9, vorzugsweise 1 bis 5, gleichen oder verschiedenen Halogenatomen wie unter Halogen definiert; beispielhaft seien genannt: Fluormethylthio, Chlormethylthio, Brommethylthio, Fluorethylthio, Chlorethylthio, Brommethylthio, Fluorpropylthio, Chlorpropylthio, Brompropylthio, Fluorbutylthio, Chlorbutylthio, Brombutylthio, Fluor-i-propylthio, Chlor-i-propylthio, Difluormethylthio, Trifluormethylthio, Dichlormethylthio, Trichlormethylthio, Difluorethylthio, Trifluorethylthio, Tetrafluorethylthio, Trichlorethylthio, Chlordifluormethylthio und Trifluorchlorethylthio.

Die hier aufgeführten Definitionen gelten in entsprechender Weise auch für die im folgenden aufgeführten bevorzugten Kombinationen von Resten.

Die erfindungsgemäßen neuen substituierten 2-[6-(Pyrimidinyl)-indol-1-yl]-acrylsäureester sind durch die Formel (I) allgemein definiert. Bevorzugt sind Verbindungen der Formel (I), bei welchen
- R¹: für geradkettiges oder verzweigtes Alkyl mit 1 bis 4 Kohlenstoffatomen steht,
- R²: für Dialkylamino mit jeweils 1 bis 4 Kohlenstoffatomen in den jeweiligen geradkettigen oder verzweigten Alkylteilen, jeweils geradkettiges oder verzweigtes Alkoxy oder Alkylthio mit 1 bis 4 Kohlenstoffatomen oder für Benzyloxy oder Benzylthio steht,
- R³ und R⁴: gleich oder verschieden sind und für Wasserstoff, Cyano, Fluor, Chlor, Brom, Methyl, Ethyl, n- oder i-Propyl, n-, i-, s- oder t-Butyl stehen,
- R⁵ und R⁶: gleich oder verschieden sind und für Wasserstoff, Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl oder Cyclohexyl stehen, oder
- R⁵ und R⁶: gemeinsam für eine Methylendioxy-, 1,3-Propandiyl- oder 1,4-Butandiylgruppierung stehen,
- R⁷: für eine der folgenden Gruppierungen steht, 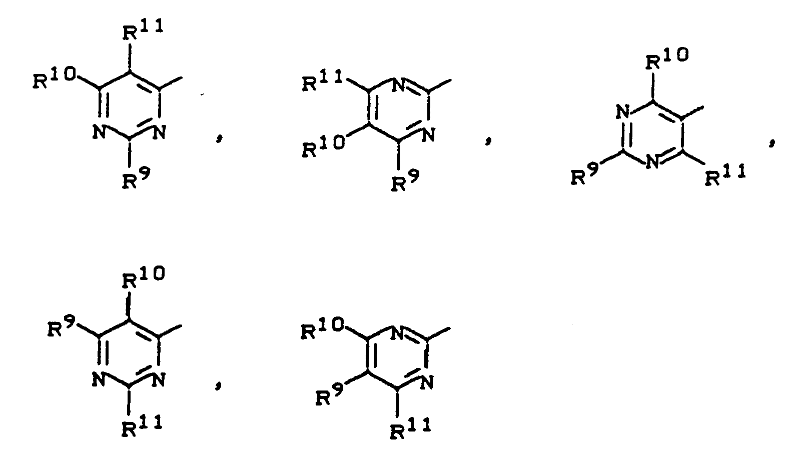 wobei
- R⁹: für Wasserstoff, jeweils geradkettiges oder verzweigtes Alkyl, Alkoxy oder Alkylthio mit jeweils 1 bis 6 Kohlenstoffatomen, Cyclopentyl, Cyclohexyl, Methylamino, Ethylamino, Dimethylamino, Diethylamino, jeweils gegebenenfalls einfach bis dreifach, gleich oder verschieden substituiertes Phenyl, Benzyl, Phenoxy, Phenylthio, Benzyloxy oder Benzylthio oder für gegebenenfalls ein- oder zweifach gleich oder verschieden substituiertes Pyrrolyl, Thienyl, Furyl, Thiazolyl, Isothiazolyl, Oxazolyl, Isoxazolyl, Pyrazolyl, Imidazolyl, Triazinyl, Pyridyl, Pyrimidyl, Pyridazinyl, Pyrazinyl, Pyridylmethyl, Thienylmethyl, Furylmethyl, Pyridyloxy, Thienyloxy, Thiazolyloxy, Pyridylmethyloxy, Thienylmethyloxy, Thienylthio, Furylthio, Pyridylmethylthio oder Thienylmethylthio steht, wobei als Substituenten jeweils Halogen, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n-oder i-Propylthio, n-, i-, s- oder t-Butylthio, Methylcarbonylamino, Ethylcarbonylamino, Methylcarbonyl, Ethylcarbonyl, n- oder i-Propylcarbonyl, n-, i-, s- oder t-Butylcarbonyl, Halogenalkyl, Halogenalkoxy oder Halogenalkylthio mit jeweils 1 oder 2 Kohlenstoffatomen und 1 bis 5 gleichen oder verschiedenen Fluor- oder Chloratomen, Dialkylamino oder Dialkylaminocarbonyl mit 1 oder 2 Kohlenstoffatomen in den einzelnen Alkylteilen, unsubstituiertes oder einfach bis zweifach, gleich oder verschieden substituiertes Phenyl oder Benzyl genannt seien, wobei als Phenyl- bzw. Benzylsubstituenten jeweils Fluor, Chlor, Methyl, Methoxy oder Phenoxy infrage kommen und
- R¹⁰ und R¹¹: gleich oder verschieden sind und für Wasserstoff, geradkettiges oder verzweigtes Alkyl mit 1 bis 4 Kohlenstoffatomen, Chlor, Brom, Cyano, Methoxycarbonyl, Ethoxycarbonyl, Dimethylaminocarbonyl, Diethylaminocarbonyl, Methylaminocarbonyl, Ethylaminocarbonyl, Methoxy, Ethoxy, Methylthio oder Ethylthio steht oder
- R¹⁰ und R¹¹: gemeinsam für eine Methylendioxy-, 1,3-Propandiyl- oder 1,4-Butandiylgruppierung stehen und
- R⁸: für Wasserstoff, Fluor, Chlor, Brom, Methyl, Ethyl, n- oder i-Propyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximininomethyl oder Ethoximinomethyl steht.

Ganz besonders bevorzugt sind Verbindungen der Formel (I), bei welchen
- R¹: für Methyl oder Ethyl steht,
- R²: für Dimethylamino, Diethylamino, Methoxy, Ethoxy, Methylthio, Ethylthio, Benzyloxy oder Benzylthio steht,
- R³: für Wasserstoff, Chlor oder Methyl steht,
- R⁴: für Wasserstoff, Chlor oder Methyl steht,
- R⁵: für Wasserstoff, Fluor, Chlor, Brom, Methyl, Ethyl, Trifluormethyl, Methoxycarbonyl oder Ethoxycarbonyl steht,
- R⁶: für Wasserstoff, Fluor, Chlor, Brom, Methyl, Ethyl, Methoxy, Ethoxy, Methylthio, Trifluormethyl, Methoxycarbonyl oder Ethoxycarbonyl steht oder
- R⁵ und R⁶: gemeinsam für eine Methylendioxy-, 1,3-Propandiyl- oder 1,4-Butandiylgruppierung stehen,
- R⁷: für eine der folgenden Gruppierungen steht 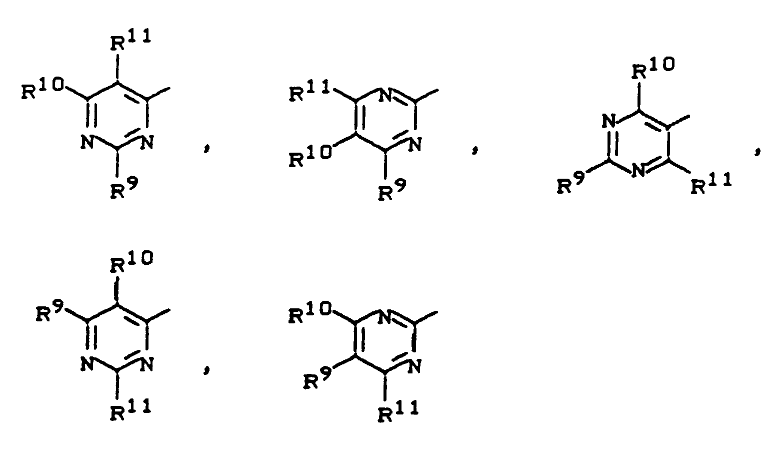 wobei
- R⁹: für Wasserstoff, Methyl, Ethyl, n- oder i-Propyl, n-, s-, i- oder t-Butyl, n- oder i-Amyl, n-Hexyl, Methoxy, Ethoxy, n- oder i- Propoxy, Methylthio, Ethylthio, n- oder i-Propylthio, Cyclohexyl, Methylamino, Ethylamino, Dimethylamino, Diethylamino, Benzyl, o-, m- oder p-Chlorbenzyl, o-, m- oder p-Methylbenzyl, gegebenenfalls einfach bis dreifach, gleich oder verschieden substituiertes Phenyl oder gegebenenfalls ein-oder zweifach, gleich oder verschieden substituiertes Pyridyl, Thienyl, Furyl, Pyrimidinyl, Pyridazinyl, Pyrazinyl oder Thiazolyl steht, wobei als Substituenten jeweils Fluor, Chlor, Methyl, Ethyl, t-Butyl, Methoxy, Ethoxy, Methylthio, Methylcarbonylamino, Methylcarbonyl, Ethylcarbonyl, Trifluormethyl, Trifluormethoxy, Trifluormethylthio, 1,3-Propandiyl, Phenyl, p-Chlorphenyl, m- oder p-Phenoxyphenyl oder Benzyl infrage kommen und
- R¹⁰ und R¹¹: unabhängig voneinander für Wasserstoff, Methyl, Ethyl, Chlor, Brom, Methoxycarbonyl oder Ethoxycarbonyl steht, oder
- R¹⁰ und R¹¹: gemeinsam für eine Methylendioxy-, 1,3-Propandiyl- oder 1,4-Butandiylgruppierung stehen und
- R⁸: für Wasserstoff, Methyl oder Ethyl steht.

Im einzelnen seien außer den bei den Herstellungsbeispielen genannten Verbindungen die folgenden substituierten 2-[6-(Pyrimidinyl)-indol-1-yl]-acrylsäureester der allgemeinen Formel (I) genannt:

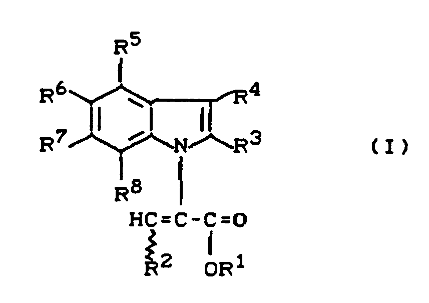

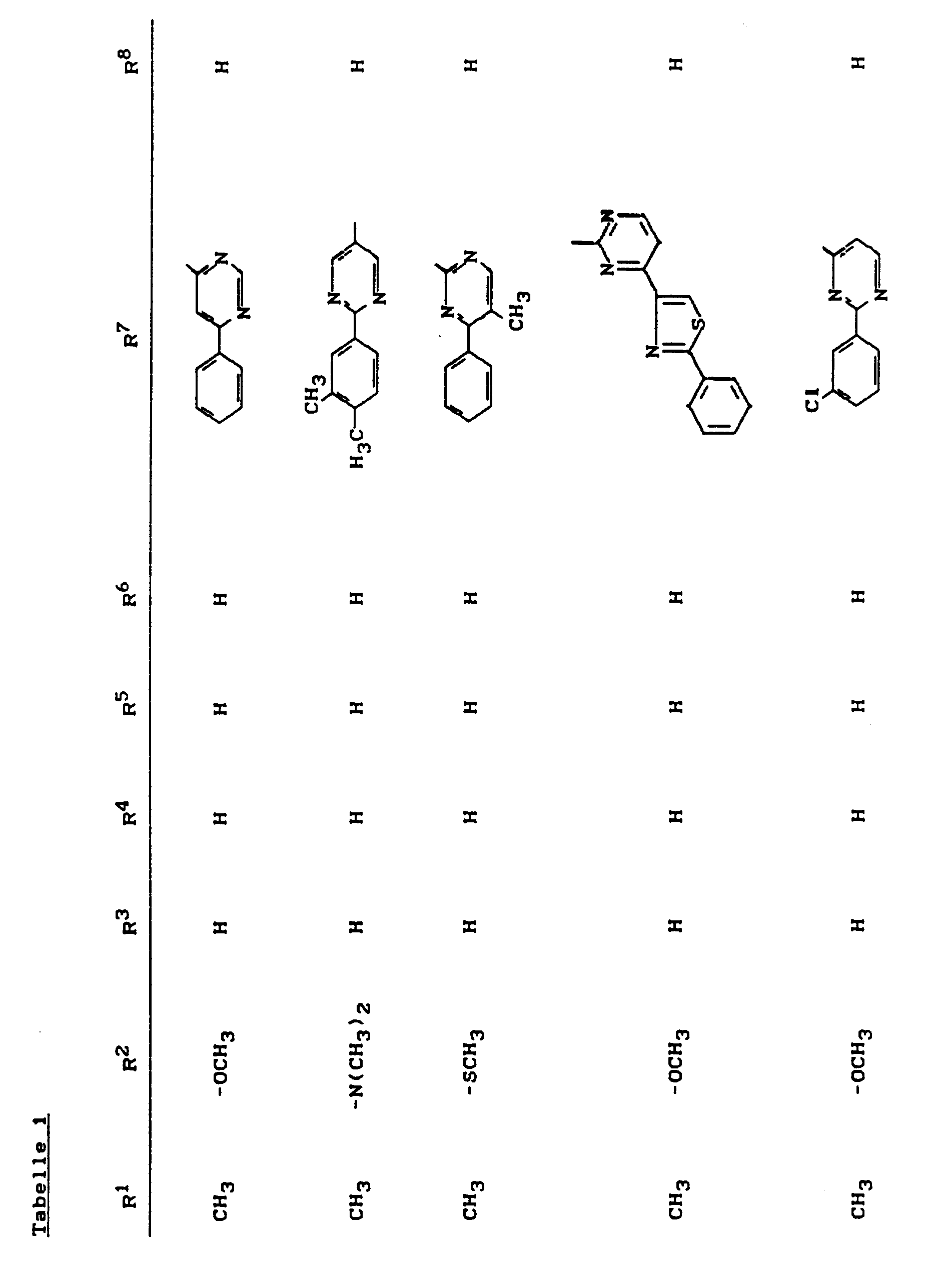

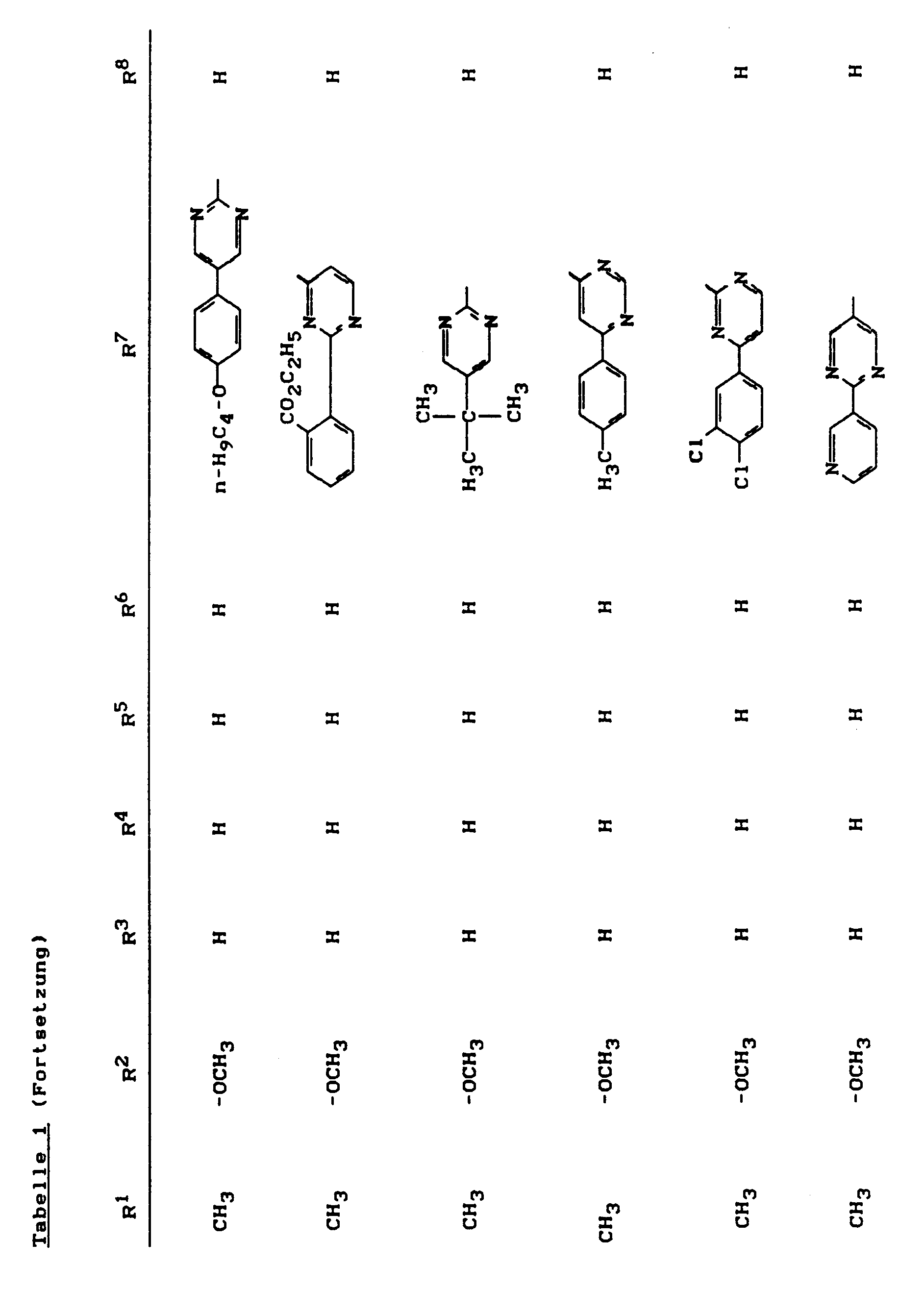

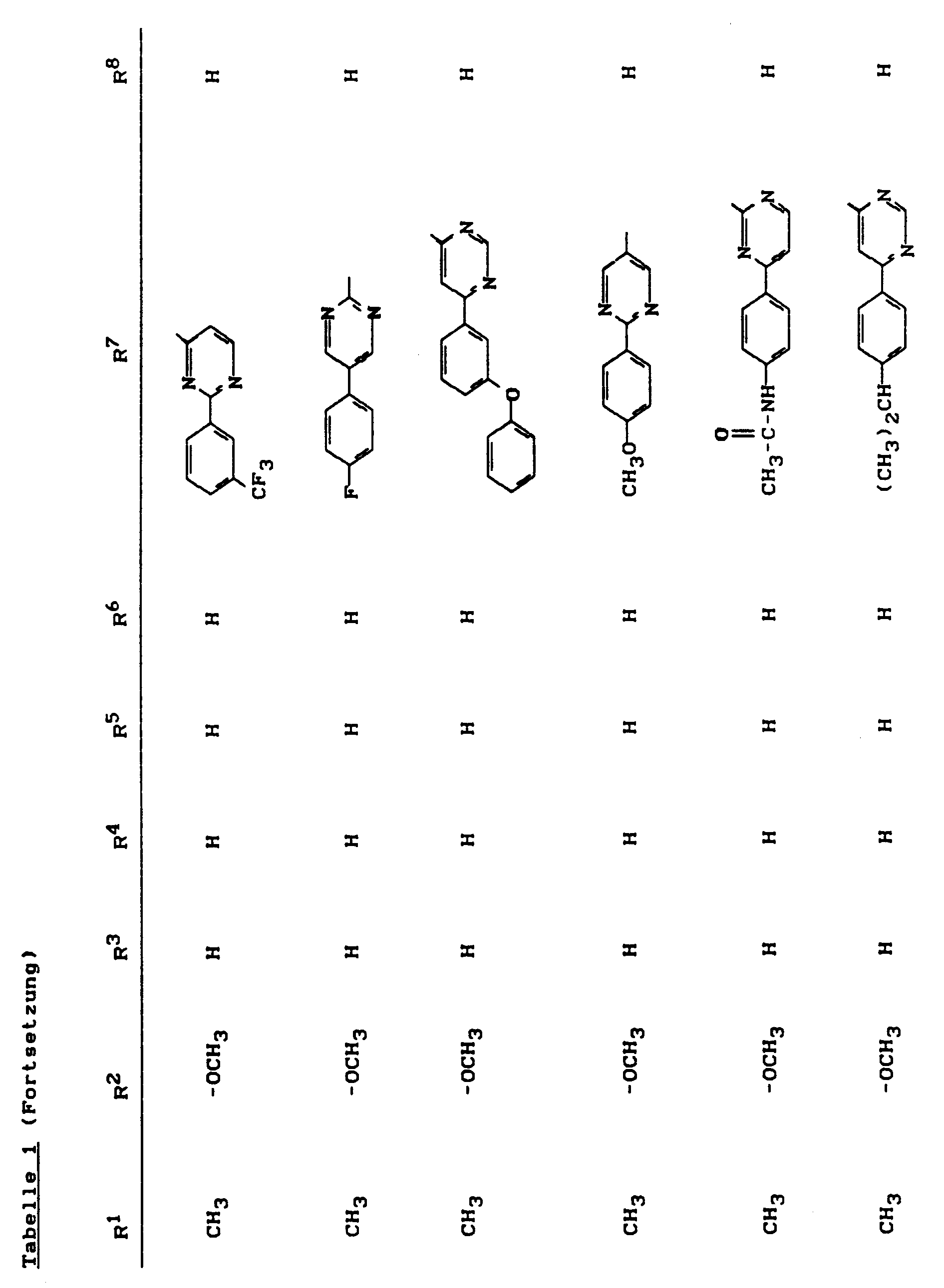

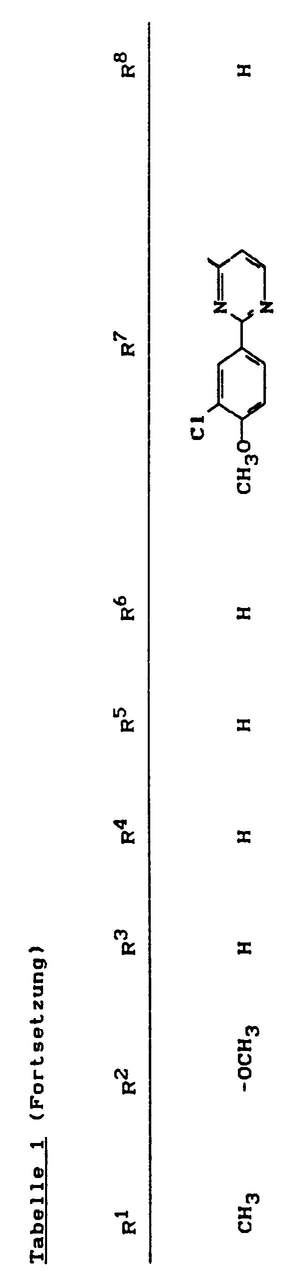

Verwendet man beispielsweise 3-Hydroxy-2-[6-[4-(phenyl)-pyrimidin-2-yl]-indol-1-yl]-acrylsäuremethylester und Dimethylsulfat als Ausgangsstoffe, so läßt sich der Reaktionsablauf des erfindungsgemäßen Verfahrens (a) durch das folgende Formelschema darstellen: 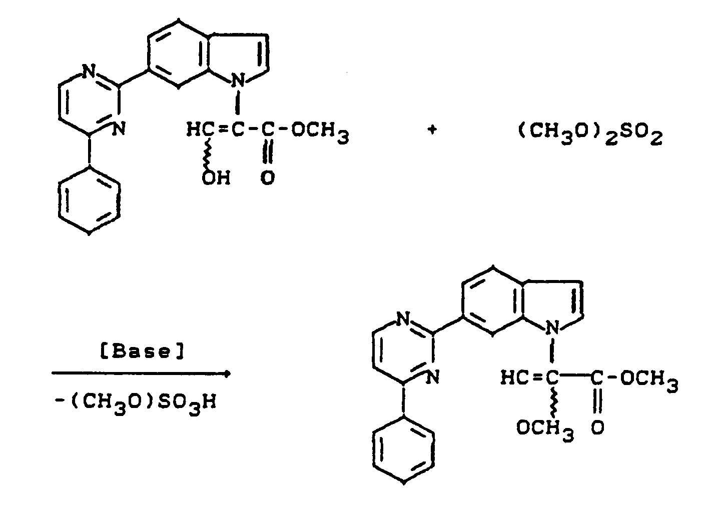
Verwendet man beispielsweise [6-[4-(Phenyl)-pyrimidin-2-yl]-indol-1-yl]-essigsäuremethylester und Dimethylformamiddimethylacetal als Ausgangsstoffe, so läßt sich der Reaktionsablauf des erfindungsgemäßen Verfahrens (b) durch das folgende Formelschema darstellen: 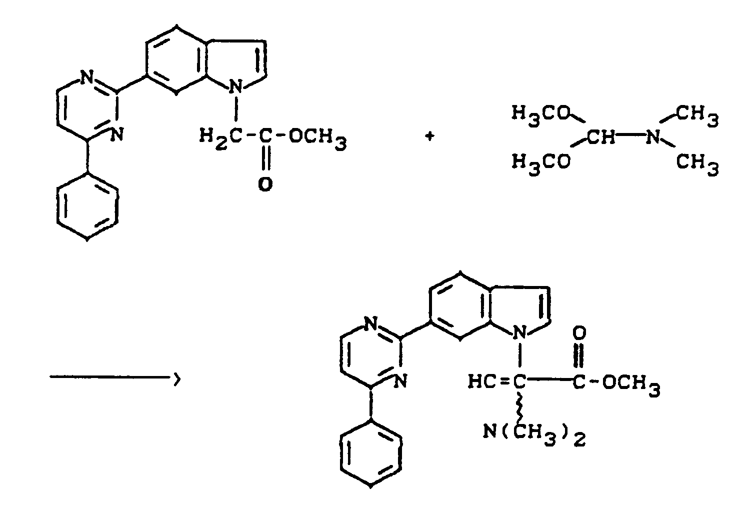
Verwendet man beispielsweise 2-Oxo-2-[6-(4-(Phenyl)-pyrimidin-2-yl]-indol-1-yl]-essigsäuremethylester und [(Methylthio)-(trimethylsilyl)]-methylen-lithium als Ausgangsverbindungen, so läßt sich der Reaktionsablauf des erfindungsgemäßen Verfahrens (c) durch das folgende Formelschema darstellen: 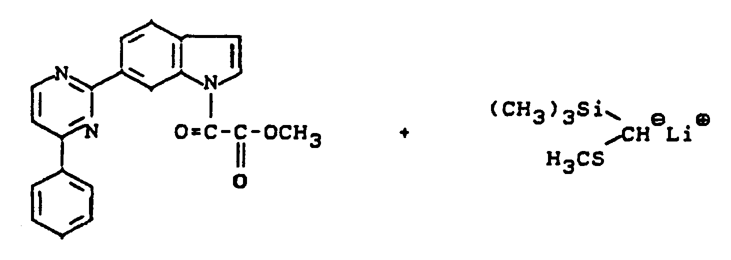 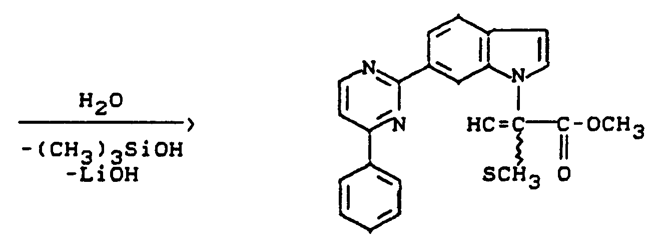
Verwendet man beispielsweise 2-[6-[4-(4-Methylphenyl)-pyrimidin-2-yl]-indol-1-yl]-3-methansulfonyloxy-acrylsäuremethylester und Methanthiol als Ausgangsstoffe, so läßt sich das erfindungsgemäße Verfahren (d) durch das folgende Formelschema darstellen: 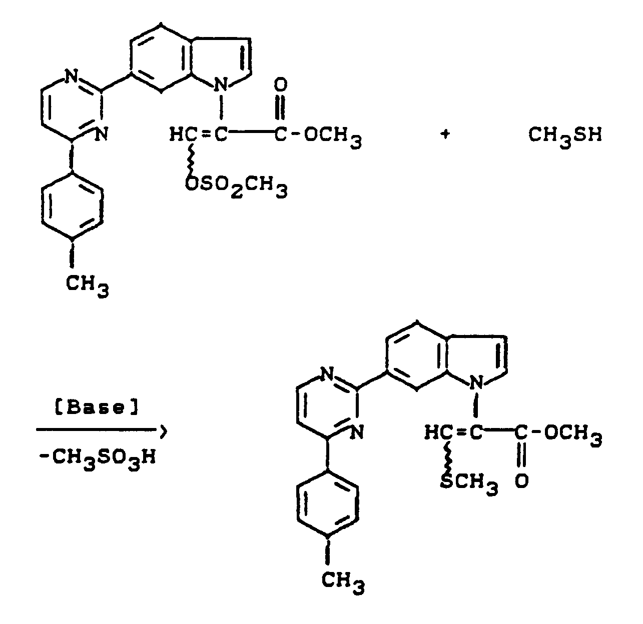
Die zur Durchführung des erfindungsgemäßen Verfahrens (a) als Ausgangsstoffe benötigten Hydroxyacrylsäureester oder deren Alkalimetallsalze sind durch die Formel (II) allgemein definiert. In dieser Formel (II) stehen R¹, R³, R⁴, R⁵, R⁶, R⁷ und R⁸ vorzugsweise für diejenigen Reste, die bereits im Zusammenhang mit der Beschreibung der erfindungsgemäßen Stoffe der Formel (I) als bevorzugt für diese Substituerten genannt wurden.
- M: steht vorzugsweise für Wasserstoff oder für ein Lithium-, Natrium- oder Kaliumkation.

Die zur Durchführung des erfindungsgemäßen Verfahrens (a) benötigten Hydroxyacrylsäureester der Formel (II) sind noch nicht bekannt und Gegenstand der Erfindung.

Man erhält sie, wenn man substituierte Essigsäureester der Formel (IV), 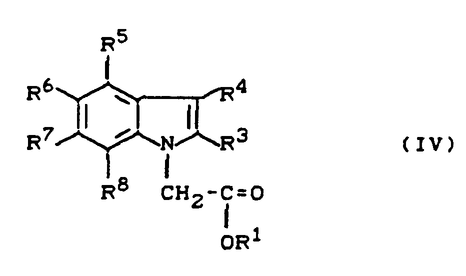
in welcher
- R¹, R³, R⁴, R⁵, R⁶, R⁷ und R⁸: die oben angegebene Bedeutung haben,
mit Ameisensäureestern der Formel (X), 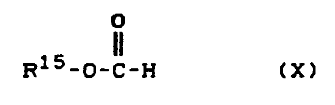
in welcher
- R¹⁵: für Alkyl, insbesondere für Methyl oder Ethyl steht,
gegebenenfalls in Gegenwart eines Verdünnungsmittels wie beispielsweise Dimethylformamid und gegebenenfalls in Gegenwart eines basischen Reaktionshilfsmittels wie beispielsweise Natriumhydrid bei Temperaturen von -20°C bis +50°C umsetzt (vgl. z.B. EP-A 274 825).

Ameisensäureester der Formel (X) sind allgemein bekannte Verbindungen der organischen Chemie.

Die zur Durchführung des erfindungsgemäßen Verfahrens (a) weiterhin als Ausgangsstoffe benötigten Alkylierungsmittel sind durch die Formel (III) allgemein definiert. In dieser Formel (III) steht R¹² vorzugsweise für diejenigen Reste, die bereits im Zusammenhang mit der Beschreibung der erfindungsgemäßen Stoffe der Formel (I) als bevorzugt für diesen Substituenten genannt wurden.
- E¹: steht für eine bei Alkylierungsmitteln übliche Abgangsgruppe, vorzugsweise für einen gegebenenfalls substituierten Alkyl-, Alkoxy- oder Arylsulfonyloxyrest, wie beispielsweise ein Methoxysulfonyloxyrest, ein Ethoxysulfonyloxyrest oder ein p-Toluolsulfonyloxyrest oder für Halogen, insbesondere für Chlor, Brom oder Iod.

Die Alkylierungsmittel der Formel (III) sind allgemein bekannte Verbindungen der organischen Chemie.

Die zur Durchführung des erfindungsgemäßen Verfahrens (b) und zur Synthese der Vorprodukte der Formel (II) als Ausgangsstoffe benötigten substituierten Essigsäureester der allgemeinen Formel (IV) sind neu und ebenfalls Gegenstand der Erfindung. In dieser Formel (IV) stehen R¹, R³, R⁴, R⁵, R⁶, R⁷ und R⁸ vorzugsweise für diejenigen Reste, die bereits im Zusammenhang mit der Beschreibung der erfindungsgemäßen Stoffe der Formel (I) als bevorzugt für diese Substituenten genannt wurden. Die substituierten Essigsäureester der allgemeinen Formel (IV) sind ebenfalls als Pflanzenschutzmittel wirksam und werden auch hinsichtlich ihrer biologischen Verwendung beansprucht.

Man erhält die Verbindungen der Formel (IV),
wenn man Indolderivate der allgemeinen Formel (XI) 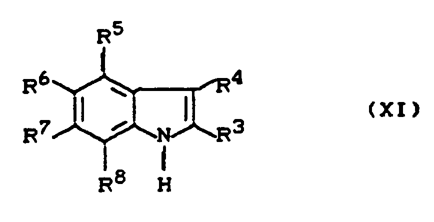
in welcher
- R³, R⁴, R⁵, R⁶, R⁷ und R⁸: die oben angegebene Bedeutung haben,
mit Essigsäurederivaten der allgemeinen Formel (XII)

E³-CH₂-COOR¹ (XII)

in welcher
- R¹: die oben angegebene Bedeutung hat und
- E³: für eine elektronenanziehende Abgangsgruppe, bevorzugt für Halogen, insbesondere für Chlor oder Brom, steht,
gegebenenfalls in Gegenwart eines Verdünnungsmittels, wie beispielsweise Acetonitril oder Aceton und gegebenenfalls in Gegenwart eines basischen Hilfsmittels, wie beispielsweise Kaliumcarbonat oder Kalium-tert.-butylat bei Temperaturen zwischen -20°C und +100°C umsetzt.

Die Essigsäurederivate der Formel (XII) sind allgemein bekannte Verbindungen der organischen Chemie.

Die Indolderivate der allgemeinen Formel (XI) sind noch nicht bekannt und Gegenstand der Erfindung. Man erhält sie jedoch nach bekannten Verfahren in analoger Weise, indem man beispielsweise Nitrobenzol-Derivate der Formel (XIII) 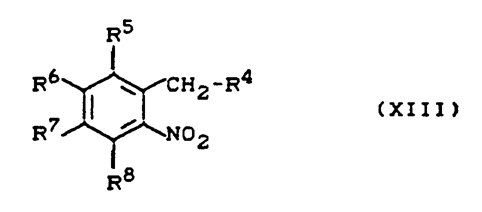
in welcher
- R⁴, R⁵, R⁶, R⁷ und R⁸: die oben angegebene Bedeutung haben,
mit Verbindungen der Formel (XIV) 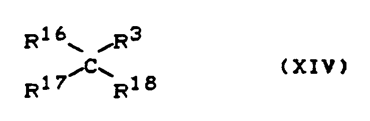
in welcher
- R³: die oben angegebene Bedeutung hat,
- R¹⁶: für Alkoxy oder Dialkylamino steht,
- R¹⁷: für Alkoxy oder Dialkylamino steht und
- R¹⁸: für Dialkylamino steht,
gegebenenfalls in Gegenwart eines Verdünnungsmittels wie beispielsweise Toluol oder Dimethylformamid bei Temperaturen zwischen 25°C und 200°C und gegebenenfalls unter einem Druck von 1 bis 100 bar zu den Verbindungen der allgemeinen Formel (XV) 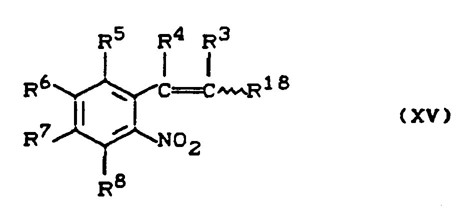
in welcher
- R³, R⁴, R⁵, R⁶, R⁷, R⁸ und R¹⁸: die oben angegebene Bedeutung haben,
umsetzt und die so erhaltenen Verbindungen der Formel (XV), gegebenenfalls nach ihrer Isolierung und/oder Reinigung, gegebenenfalls in Gegenwart eines Verdünnungsmittels, wie beispielsweise Methanol, Ethanol, Tetrahydrofuran oder Dioxan gegebenenfalls in Gegenwart eines Inertgases, wie beispielsweise Stickstoff, mit üblichen Reduktionsmitteln, wie beispielsweise Wasserstoff, in Gegenwart eines geeigneten Katalysators, wie beispielsweise Raney-Nickel und einem Druck zwischen 1 und 200 bar, bei Temperaturen zwischen -20°C und +200°C cyclisiert.

Die Verbindungen der Formel (XV) sind neu und ebenfalls Gegenstand der Erfindung.

Die Verbindungen der allgemeinen Formel (XIV) sind allgemein bekannte Verbindungen der organischen Chemie (vgl. Tetrahedron 35, 1675 (1979)).

Die Nitrobenzolderivate der Formel (XIII) 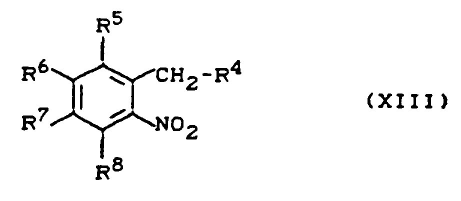
in welcher
- R⁴, R⁵, R⁶, R⁷ und R⁸: die oben angegebene Bedeutung haben, sind neu.

Man erhält die Nitrobenzolderivate der Formel (XIII) nach einem der im folgenden beschriebenen Verfahren:
a) Man erhält Nitrobenzolderivate der allgemeinen Formel (XIII-a) 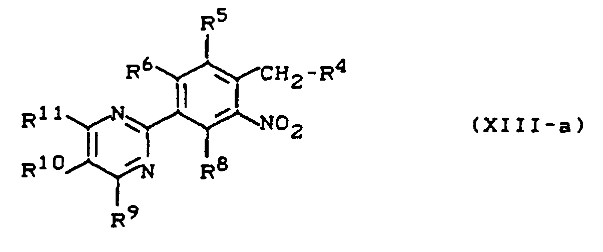 in welcher
   - R⁴, R⁵, R⁶, R⁸, R⁹, R¹⁰ und R¹¹: die oben angegebene Bedeutung haben,
   wenn man Verbindungen der Formel (XVI) 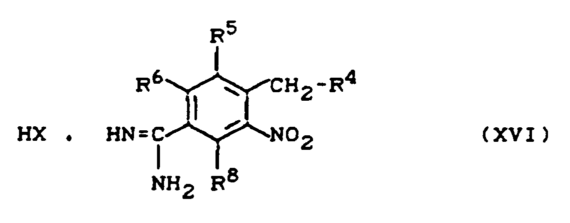 in welcher
   - R⁴, R⁵, R⁶ und R⁸: die oben angegebene Bedeutung haben, und
   - HX: für das Äquivalent einer anorganischen Säure wie beispielsweise Chlorwasserstoffsäure oder einer organischen Säure wie beispielsweise Oxalsäure steht,
   mit Enaminonen der Formel (XVII) 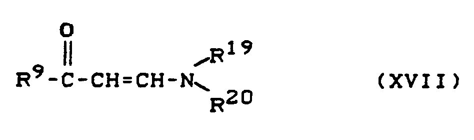 in welcher
   - R⁹: die oben angegebene Bedeutung hat, und
   - R¹⁹ und R²⁰: gleich oder verschieden sind und vorzugsweise für geradkettiges oder verzweigtes Alkyl mit 1 bis 6 Kohlenstoffatomen, insbesondere für geradkettiges oder verzweigtes Alkyl mit 1 bis 4 Kohlenstoffatomen stehen,
   gegebenenfalls in Gegenwart eines Verdünnungsmittels wie beispielsweise Methanol oder Ethanol und gegebenenfalls in Gegenwart einer Base wie beispielsweise Natriummethanolat oder Natriumethanolat bei Temperaturen zwischen +20°C und +200°C umsetzt.
b) Man erhält Nitrobenzolderivate der allgemeinen Formel (XIII-b) 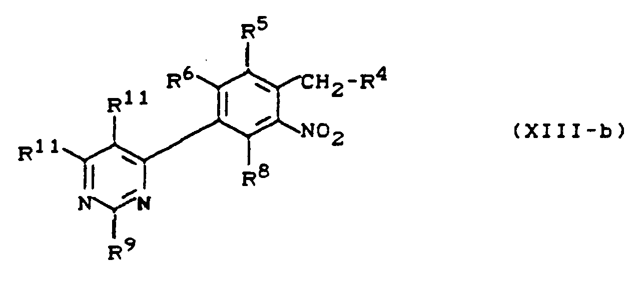 in welcher
   - R⁴, R⁵, R⁶, R⁸, R⁹, R¹⁰ und R¹¹: die oben angegebene Bedeutung haben,
   wenn man Verbindungen der Formel (XVIII) 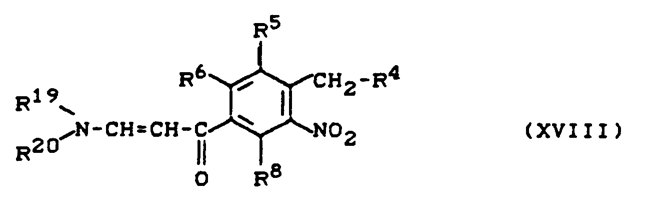 in welcher
   - R⁴, R⁵, R⁶, R⁸, R¹⁹ und R²⁰: die oben angegebene Bedeutung haben,
   mit Amidinderivaten der Formel (XIX) 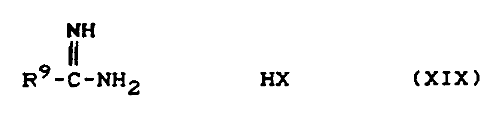 in welcher
   - R⁹: die oben angegebene Bedeutung hat und
   - HX: für das Äquivalent einer anorganischen Säure wie beispielsweise Chlorwasserstoffsäure oder einer organischen Säure wie beispielsweise Oxalsäure steht,
   gegebenenfalls in Gegenwart eines Verdünnungsmittels wie beispielsweise Methanol oder Ethanol und gegebenenfalls in Gegenwart einer Base wie beispielsweise Natriummethanolat oder Natriumethanolat bei Temperaturen zwischen +20°C und +200°C umsetzt.
c) Man erhält die Nitrobenzolderivate der allgemeinen Formeln (XIII-c), (XIII-d) und (XIII-e) 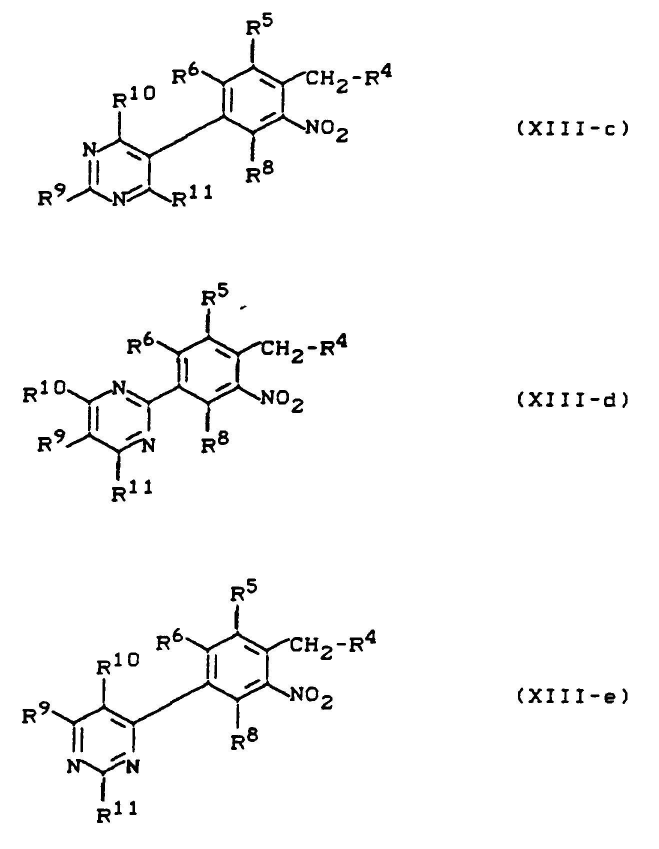 in welchen
   - R⁴, R⁵, R⁶, R⁸, R⁹, R¹⁰ und R¹¹: die oben angegebene Bedeutung haben,
   wenn man Nitrohalogenaromaten der allgemeinen Formel (XX) 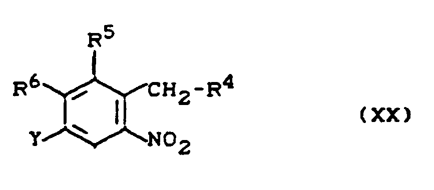 in welcher
   - R⁴, R⁵ und R⁶: die oben angegebene Bedeutung haben, und
   - Y: für Halogen, vorzugsweise für Brom oder Jod steht,
   entweder
   α) mit Boronsäuren der Formel (XXI) 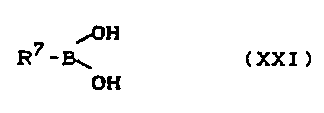 in welcher
      - R⁷: die oben angegebene Bedeutung hat, oder
   β) mit Zinnverbindungen der Formel (XXII)

      R⁷-Sn(R²¹)₃ (XXII)

      in welcher
      - R⁷: die oben angegebene Bedeutung hat, und
      - R²¹: vorzugsweise für geradkettiges oder verzweigtes Alkyl mit 1 bis 6 Kohlenstoffatomen, insbesondere für geradkettiges oder verzweigtes Alkyl mit 1 bis 4 Kohlenstoffatomen steht,
   auf bekannte weise (vgl. Nachr. Chem. Tech. Lab. 36, 1324 (1958) in einer Zweiphasenreaktion in Gegenwart eines Verdünnungsmittels wie beispielsweise Benzol oder Toluol und in Gegenwart einer Base wie beispielsweise wäßriger Natriumcarbonat- oder Natriumhydrogencarbonat-Lösung sowie in Gegenwart eines Katalysators wie beispielsweise Tetrakisphenylphosphinpalladium (0), Palladiumdichlorid/Triphenylphosphin oder Bis-(triphenylphosphin)palladiumdichlorid bei Temperaturen zwischen +20°C und +200°C umsetzt.

Die Verbindungen der Formel (XVI) und die Amidinderivate der Formel (XIX) sind bekannt und/oder können in Analogie zu bekannten Verfahren hergestellt werden (vgl. Houben-Weyl-Müller, "Methoden der organischen Chemie", Vol. XI/2, S. 38 ff., Thieme Verlag Stuttgart (1958)).

Enaminone der Formel (XVII) und Verbindungen der Formel (XVIII) sind bekannt und/oder können in Analogie zu bekannten Verfahren hergestellt werden (vgl. Chem. Ber. 97, 3397 (1964)).

Boronsäuren der Formel (XXI) sind bekannt und/oder können in Analogie zu bekannten Verfahren hergestellt werden (vgl. Adv. Chem. Ser. 102 (1959)).

Zinnverbindungen der Formel (XXII) sind ebenfalls bekannt und/oder nach bekannten Verfahren herstellbar (vgl. Tetrahedron Lett. 4407 (1986)).

Die zur Durchführung des erfindungsgemäßen Verfahrens (b) weiterhin als Ausgangsstoffe benötigten Formamide und deren Derivate sind durch die Formeln (Va) und (Vb) allgemein definiert. In diesen Formeln (Va) und (Vb) steht R²⁻² vorzugsweise für Dialkylamino mit jeweils 1 bis 6, insbesondere mit 1 bis 4 Kohlenstoffatomen in den einzelnen geradkettigen oder verzweigten Alkylteilen. R²⁻² steht ganz besonders bevorzugt für Dimethylamino oder Diethylamino.
- R¹³ und R¹⁴: stehen vorzugsweise unabhängig voneinander jeweils für geradkettiges oder verzweigtes Alkoxy mit 1 bis 4 Kohlenstoffatomen insbesondere für Methoxy oder Ethoxy oder für einen Dialkylaminorest, mit jeweils 1 bis 6 insbesondere mit 1 bis 4 Kohlenstoffatomen in den einzelnen geradkettigen oder verzweigten Alkylteilen.

Die Formamide der Formel (Va) und deren Derivate der Formel (Vb) sind allgemein bekannte Verbindungen der organischen Chemie.

Die zur Durchführung des erfindungsgemäßen Verfahrens (c) als Ausgangsstoffe benötigten Ketocarbonsäurederivate sind durch die Formel (VI) allgemein definiert.

In dieser Formel (VI) stehen R¹, R³, R⁴, R⁵, R⁶, R⁷ und R⁸ vorzugsweise für diejenigen Reste, die bereits im Zusammenhang mit der Beschreibung der erfindungsgemäßen Stoffe der Formel (I) als bevorzugt für diese Substituenten genannt wurden.

Die Ketocarbonsäurederivate der Formel (VI) sind neu und ebenfalls Gegenstand der Erfindung. Man erhält sie jedoch in Analogie zu bekannten Verfahren, indem man beispielsweise Oxalester der Formel (XXIII) 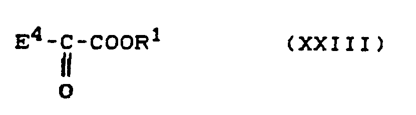
in welcher
- R¹: die oben angegebene Bedeutung hat und
- E⁴: für Alkoxy oder Halogen, insbesondere für Methoxy, Ethoxy oder Chlor steht,
mit Indolderivaten der Formel (XI) 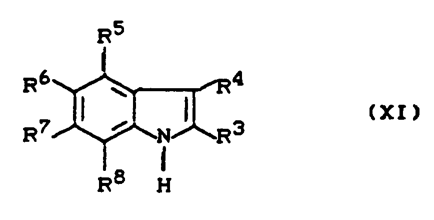
in welcher
- R³, R⁴, R⁵, R⁶, R⁷ und R⁸: die oben angegebene Bedeutung
gegebenenfalls in Gegenwart eines Verdünnungsmittels, wie beispielsweise Dichlormethan oder Tetrahydrofuran und gegebenenfalls in Gegenwart einer Base, wie beispielsweise n-Butyllithium, Natriumhydrid, Kalium-t-butylat, Triethylamin oder Pyridin bei Temperaturen zwischen -80°C und +80°C umsetzt (vgl. DE-OS 3807232).

Die zur Durchführung des erfindungsgemäßen Verfahrens (c) weiterhin als Ausgangsstoffe benötigten metallorganischen Verbindungen sind durch die Formel (VII) allgemein definiert, In dieser Formel (VII) steht R²⁻³ vorzugsweise für diejenigen Reste, die bereits im Zusammenhang mit der Beschreibung der erfindungsgemäßen Stoffe der Formel (I) als bevorzugt für diesen Substituenten genannt wurden.

Die metallorganischen Verbindungen der Formel (VII) sind bekannt (vgl. z.B. J.Org.Chem. 33, 780 [1968]; J.Org.Chem. 37, 939 [1972]).

Die zur Durchführung des erfindungsgemäßen Verfahrens (d) als Ausgangsstoffe benötigten substituierten Acrylsäureester sind durch die Formel (VIII) allgemein definiert, In dieser Formel (VIII) stehen R¹, R³, R⁴, R⁵, R⁶, R⁷ und R⁸ vorzugsweise für diejenigen Reste, die bereits im Zusammenhang mit der Beschreibung der erfindungsgemäßen Stoffe der Formel (I) als bevorzugt für diese Substituenten genannt wurden.
- E²: steht vorzugsweise für einen geeigneten Acyloxy- oder Sulfonyloxyrest, insbesondere für einen Acetoxy-, einen Methansulfonyloxy- oder einen p-Toluolsulfonyloxyrest.

Die substituierten Acrylsäureester der Formel (VIII) sind noch nicht bekannt.

Man erhält sie, wenn man Hydroxyacrylsäureester der Formel (II), 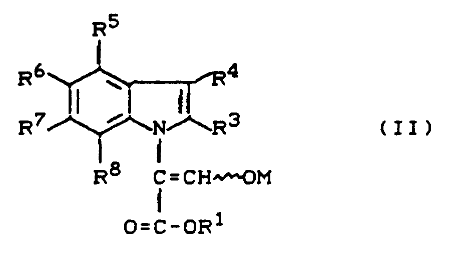
in welcher
- M, R¹, R³, R⁴, R⁵, R⁶, R⁷ und R⁸: die oben angegebene Bedeutung haben,
mit Säurechloriden der Formel (XXIV)

R²²-Cl (XXIV)

in welcher
- R²²: für einen Acyl- oder Sulfonylrest, insbesondere für einen Acetyl-, einen Methansulfonyl- oder einen p-Toluolsulfonylrest steht,
gegebenenfalls in Gegenwart eines Verdünnungsmittels wie beispielsweise Dichlormethan und gegebenenfalls in Gegenwart eines Säurebindemittels wie beispielsweise Triethylamin oder Pyridin bei Temperaturen von -20°C bis +120°C umsetzt.

Säurechloride der Formel (XXIV) sind allgemein bekannte Verbindungen der organischen Chemie.

Die zur Durchführung des erfindungsgemäßen Verfahrens (d) weiterhin als Ausgangsstoffe benötigten Thiole sind durch die Formel (IX) allgemein definiert. In dieser Formel (IX) steht R²⁻³ vorzugsweise für diejenigen Reste, die bereits im Zusammenhang mit der Beschreibung der erfindungsgemäßen Stoffe der Formel (I) als bevorzugt für diesen Substituenten genannt wurden.

Die Thiole der Formel (IX) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform oder Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Sulfoxide, wie Dimethylsulfoxid.

Das erfindungsgemäße Verfahren (a) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-C₁₃/C₁₅-alkylammoniumchlorid, Dibenzyl-dimethyl-ammoniummethylsulfat, Dimethyl-C₁₂/C₁₄-alkyl-benzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxyethoxy)-ethyl]-amin.

Das erfindungsgemäße Verfahren (a) wird vorzugsweise in Gegenwart eines geeigneten basischen Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen von - 30 °C bis + 120 °C, vorzugsweise bei Temperaturen von - 20 °C bis + 60 °C.

Zur Durchführung des erfindungsgemäßen Verfahrens (a) setzt man pro Mol an 3-Hydroxyacrylsäureester oder eines entsprechenden Alkalimetallsalzes der Formel (II) im allgemeinen 1.0 bis 10.0 Mol, vorzugsweise 1.0 bis 5.0 Mol an Alkylierungsmittel der Formel (III) und gegebenenfalls 1.0 bis 5.0 Mol, vorzugsweise 1.0 bis 2.0 Mol an Reaktionshilfsmittel ein.
Dabei ist es auch möglich, die als Ausgangsverbindungen zur Durchführung des erfindungsgemäßen Verfahrens (a) benötigten 3-Hydroxyacrylsäureester oder deren Alkalimetallsalze der Formel (II) in einer vorgelagerten Reaktion direkt im Reaktionsgefäß herzustellen und direkt aus dem Reaktionsgemisch heraus ohne Isolierung mit dem Alkylierungsmittel der Formel (III) gemäß dem erfindungsgemäßen Verfahren (a) weiter umzusetzen ("Eintopfverfahren").
Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff oder Ether, wie Diethylether, Dioxan, Tetrahydrofuran oder Ethylenglykoldimethyl- oder -diethylether.

Es ist jedoch auch möglich, das erfindungsgemäße Verfahren (b) ohne Zusatz eines Verdünnungsmittels durchzuführen.

Die Reaktionstemperaturen können bei der Durchführung des erfindungsgemäßen Verfahrens (b) in einem größeren Bereich variiert werdend Im allgemeinen arbeitet man bei Temperaturen von - 20 °C bis + 200 °C, vorzugsweise bei Temperaturen von 0 °C bis 150 °C.

Zur Durchführung des erfindungsgemäßen Verfahrens (b) setzt man pro Mol an substituiertem Essigsäureester der Formel (IV) im allgemeinen 1.0 bis 30.0 Mol, vorzugsweise 1.0 bis 15.0 Mol an Formamid der Formel (Va) oder eines entsprechenden Derivates der Formel (Vb) ein.

Die Reaktionsdurchführung, Aufarbeitung und Isolierung der Reaktionsprodukte erfolgt nach allgemein üblichen Methoden (vgl. hierzu auch G. Mathieu; J. Weill-Raynal "Formation of C-C-Bonds", Vol. I; p. 229-244; Thieme Verlag Stuttgart 1973).

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische Kohlenwasserstoffe, wie beispielsweise Benzin, Toluol, Xylol, Petrolether, Hexan oder Cyclohexan oder Ether, wie Diethylether, Dioxan, Tetrahydrofuran oder Ethylenglykoldimethyl- oder -diethylether.

Die Reaktionstemperaturen können bei der Durchführung des erfindungsgemäßen Verfahrens (c) in einem größeren Bereich variiert werden. Im allgemeinen arbeitet man bei Temperaturen von -100°C bis +100°C, vorzugsweise bei Temperaturen von -80°C bis +50°C.

Zur Durchführung des erfindungsgemäßen Verfahrens (c) setzt man pro Mol an Ketocarbonsäurederivat der Formel (VI) im allgemeinen 1.0 bis 1.5 Mol, vorzugsweise 1.0 bis 1.2 Mol an metallorganischer Verbindung der Formel (VII) ein.
Die Reaktionsdurchführung, Aufarbeitung und Isolierung der Reaktionsprodukte erfolgt nach bekannten Verfahren (vgl. z.B. J.Org.Chem. 33, 780 [1968]; J.Org.Chem. 37, 939 [1972]).

Als Verdünnungsmittel zur Durchführung des erfindungsgemäßen Verfahrens (d) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform oder Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (d) wird vorzugsweise in Gegenwart eines geeigneten Reaktionshilfsmittels durchgeführt. Als solche kommen alle üblichen anorganischen oder organischen Basen infrage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat oder tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (d) in einem größeren Bereich variiert werden. Im allgemeinen arbeitet man bei Temperaturen von -20°C bis 180°C, vorzugsweise bei Temperaturen von 0°C bis 150°C.

Das erfindungsgemäße Verfahren kann in Abhängigkeit vom Siedepunkt der verwendeten Reaktionspartner, beispielsweise beim Einsatz von niedrigsiedenden Thiolen der Formel (IX) gegebenenfalls auch unter Druck durchgeführt werden.

Vorzugsweise arbeitet man dann bei dem Druck, der sich beim Erhitzen auf die erforderliche Reaktionstemperatur unter den Reaktionsbedingungen einstellt.

Zur Durchführung des erfindungsgemäßen Verfahrens (d) setzt man pro Mol an substituiertem Acrylsäureester der Formel (VIII) im allgemeinen 1.0 bis 20.0 Mol, vorzugsweise 1.0 bis 5.0 Mol an Thiol der Formel (IX) und gegebenenfalls 1.0 bis 5.0 Mol, vorzugsweise 1.0 bis 1.5 Mol an Reaktionshilfsmittel ein.
Die Reaktionsdurchführung, Aufarbeitung und Isolierung der Reaktionsprodukte erfolgt nach allgemein üblichen Methoden.

Die erfindungsgemäßen Wirkstoffe der Formel (I) und der Formel (IV) weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide und Insektizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe der Formeln (I) und (IV) mit besonders gutem Erfolg zur protektiven Bekämpfung der Reisfleckenkrankheit (Pyricularia oryzae) sowie von plasmopora an Reben. Außerdem zeigen die erfindungsgemäßen Wirkstoffe sehr gute fungizide Wirkung gegen septoria nodorum, cochliobolus sativus, pyrenophora teres und fusarium nivale.

Darüber hinaus zeigen die erfindungsgemäßen Wirkstoffe der Formeln (I) und (IV) außerdem eine fungizide Wirkung gegen Oomyceten, Venturia-Arten an Äpfeln, Botrytis und Pellicularia sasakii an Reis sowie eine breite und gute in vitro-Wirkung.

Desweiteren eignen sich die Wirkstoffe der Formel (I) zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.

Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.

Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Globedera ssp., Meloidogyne spp., Aphelenchoides spp., Longidorus spp., Xiphinema spp., Trichodorus spp..

Die erfindungsgemäßen Wirkstoffe der Formel (I) zeichnen sich durch eine hervorragende insektizide Wirksamkeit, insbesondere beim Einsatz gegen Käferlarven, wie z.B. Phaedon choleariae, Plutella xylostella und Spodoptera frugiperda, gegen Zikaden, wie z.B. Nephotettix, sowie gegen Blattläuse, wie z.B. Myzus persicae und gegen Spinnmilben wie z.B. Tetranychus urticae. Außerdem zeigen die erfindungsgemäßen Wirkstoffe sehr gute ovizide Wirkung.

Die Wirkstoffe der Formeln (I) und (IV) können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage:
z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen 0,1 bis 95 Gewichtsprozent Wirkstoff, vorzugsweise 0,5 bis 90 %.

Die erfindungsgemäßen Wirkstoffe der Formeln (I) und (IV) können in den Formulierungen in Mischung mit anderen bekannten Wirkstoffen vorliegen wie Fungizide, Insektizide, Akarizide und Her-bizide sowie in Mischungen mit Düngemitteln und Wachstumsregulatoren.

Die Wirkstoffe der Formeln (I) und (IV) können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

### Herstellungsbeispiele:

### Beispiel 1:

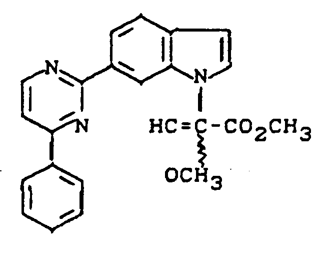

### (Verfahren (a)) ("Eintopf"-Variante)

Zu 1,71 g (57 mmol) Natriumhydrid (80%ig in Paraffin), suspendiert in 15 ml Dimethylformamid, läßt man unter Rühren und Kühlen, bei einer Temperatur von 0°C bis 5°C, eine Mischung aus 8,58 g (25 mmol) [6-[4-(Phenyl)-pyrimidin-2-yl]-indol-1-yl]-essigsäuremethylester und 28,6 ml Ameisensäuremethylester in 15 ml Dimethylformamid langsam eintropfen. Es wird 2 Stunden bei 0°C nachgerührt und das Gemisch dann unter intensivem Rühren bei der gleichen Temperatur mit 7,5 ml (79 mmol) Dimethylsulfat versetzt. Man rührt eine weitere Stunde nach und läßt dabei auf Raumtemperatur kommen; das Reaktionsgemisch wird sodann mit überschüssiger gesättigter Natriumhydrogencarbonatlösung versetzt und mit Essigester extrahiert.

Die vereinigten organischen Phasen werden mit Wasser gewaschen, über Natriumsulfat getrocknet und nach Filtration im Vakuum vom Lösungsmittel befreit. Der Rückstand wird säulenchromatographisch gereinigt (Laufmittel: Petrolether/Essigester 2:1).

Man erhält 7,5 g (78% der Theorie) an 3-Methoxy-2-[6-[4-(phenyl)-pyrimidin-2-yl]-indol-1-yl]-acrylsäuremethylester als E/Z-Isomerengemisch vom Schmelzpunkt 85°C.

### Beispiel 2:

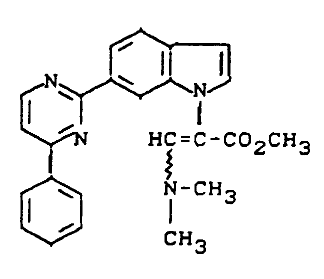

### (Verfahren (b))

Eine Mischung aus 1 g (2,9 mmol) [6-[4-(Phenyl)-pyrimidin-2-yl]-indol-1-yl]-essigsäuremethylester und 4 g Dimethylformamiddimethylacetal wird unter Rühren für 30 Stunden am Rückfluß zum Sieden erhitzt. Man läßt abkühlen, engt ein, verrührt den Rückstand mit Diisopropylether und saugt ab, Der Feststoff wird mit wenig Diisopropylether gewaschen und getrocknet.

Man erhält 1,1 g (95% der Theorie) an 3-Dimethylamino-2-[6-[4-(phenyl)-pyrimidin-2-yl]-indol-1-yl]-acrylsäuremethylester als E/Z-Gemisch vom Schmelzpunkt 168°C.

Analog zu den Herstellungsbeispielen 1 und 2 und gemäß den allgemeinen Angaben zu den erfindungsgemäßen Verfahren (a), (b), (c) und (d) können die in Tabelle 2 aufgeführten Verbindungen der Formel (I) hergestellt werden.

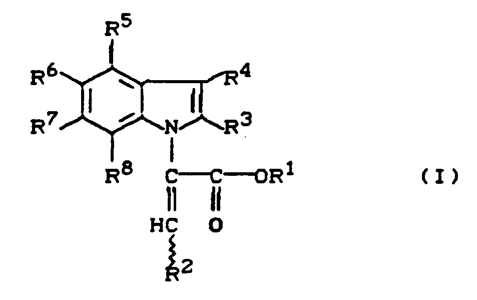

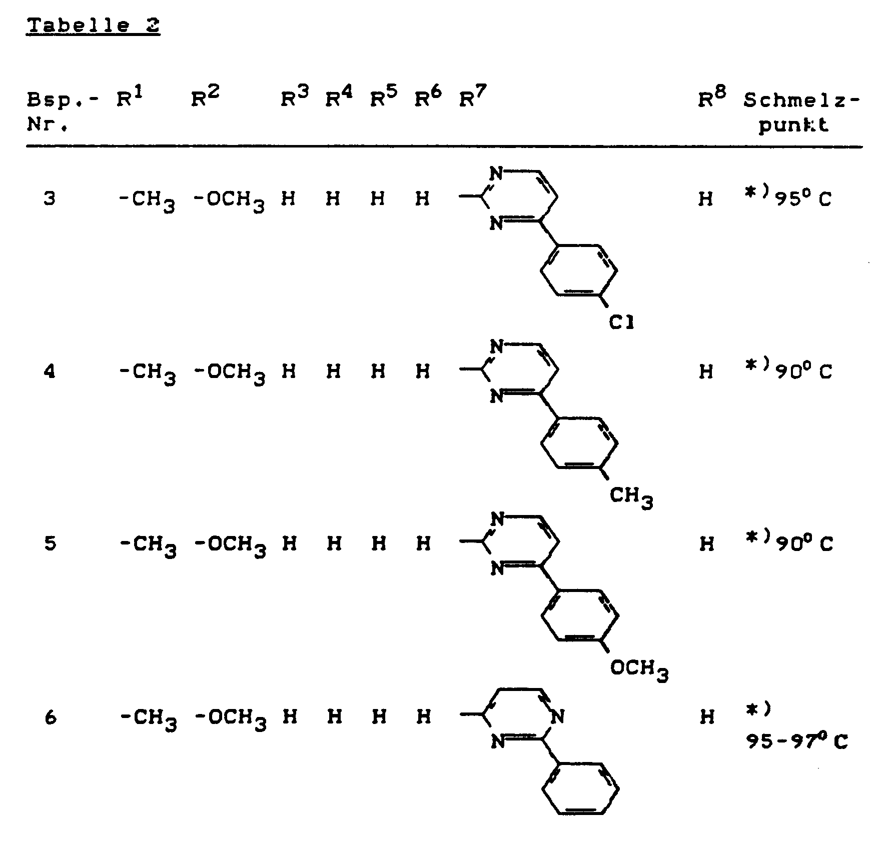

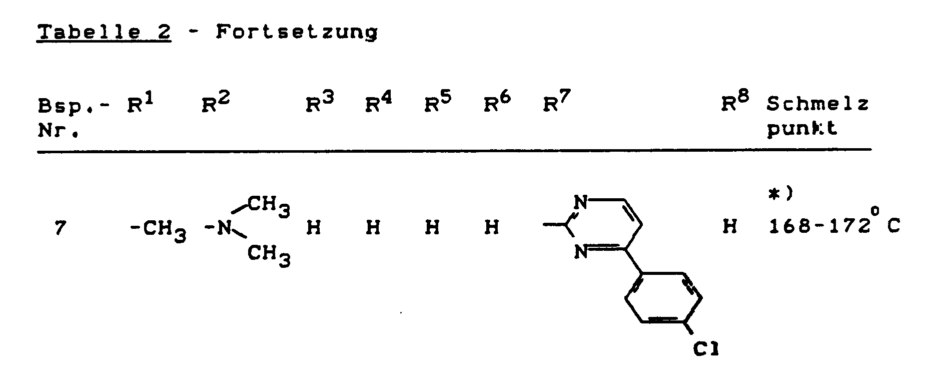

### Herstellung der Ausgangsverbindungen

### Beispiel (IV-1)

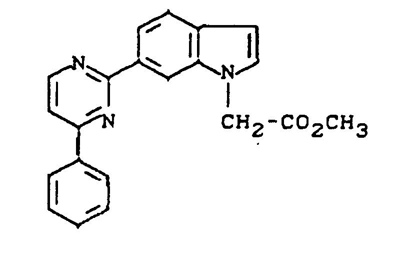

Man erhitzt eine Mischung aus 16,0 g (59 mmol) 6-[4-(Phenyl)-pyrimidin-2-yl]-indol, 17,5 g (114 mmol) Bromessigsäuremethylester, 23,5 g Kaliumcarbonat und 130 ml Acetonitril 6 Stunden am Rückfluß zum Sieden und verfolgt den Reaktionsverlauf dünnschichtchromatographisch. Nach Beendigung der Reaktion läßt man abkühlen, verteilt das Gemisch zwischen Wasser und Essigester, trocknet die organische Phase über Natriumsulfat und engt ein. Der Rückstand wird säulenchromatographisch gereinigt (Laufmittel: Petrolether/Essigester 1:1).

Man erhält 12,9 g (53% der Theorie) an [6-[4-(Phenyl)-pyrimidin-2-yl]-indol-1-yl]-essigsäuremethylester vom Schmelzpunkt 111°C.

Analog zum Herstellungsbeispiel (IV-1) können die in Tabelle 3 aufgeführten Verbindungen der Formel (IV) erhalten werden.

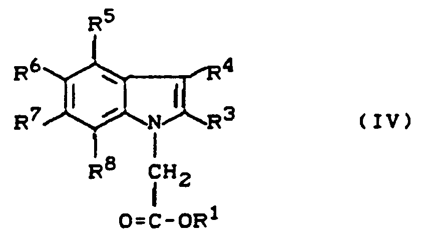

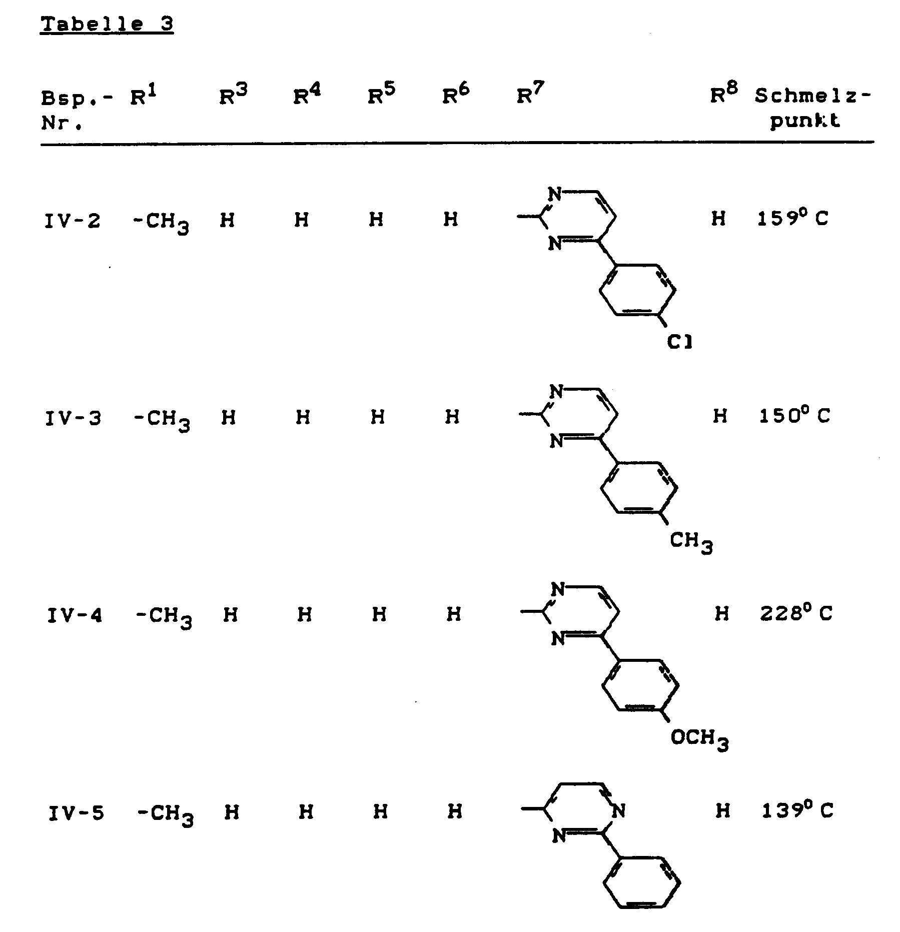

### Beispiel (XI-1)

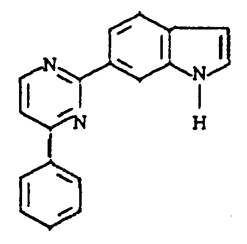

In einem Autoklaven wird eine Lösung von 26,3 g (76 mmol) β-Dimethylamino-4-[4-(phenyl)-pyrimidin-2-yl]-2-nitro-styrol in 200 ml Tetrahydrofuran unter Zusatz von 5 g Raney-Nickel bei einem Wasserstoffdruck von 50 bar und einer Temperatur von 70°C 5 Stunden lang hydriert. Der Katalysator wird durch Filtration abgetrennt, das Filtrat eingeengt und der Rückstand säulenchromatographisch gereinigt (Laufmittel: Essigester).

Man erhält 19,8 g (66% der Theorie) an 6-[4-(Phenyl)-pyrimidin-2-yl]-indol vom Schmelzpunkt 160°C.

Analog zum Herstellungsbeispiel (XI-1) können die in Tabelle 4 aufgeführten Verbindungen der Formel (XI) erhalten werden.

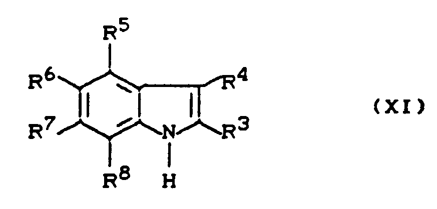

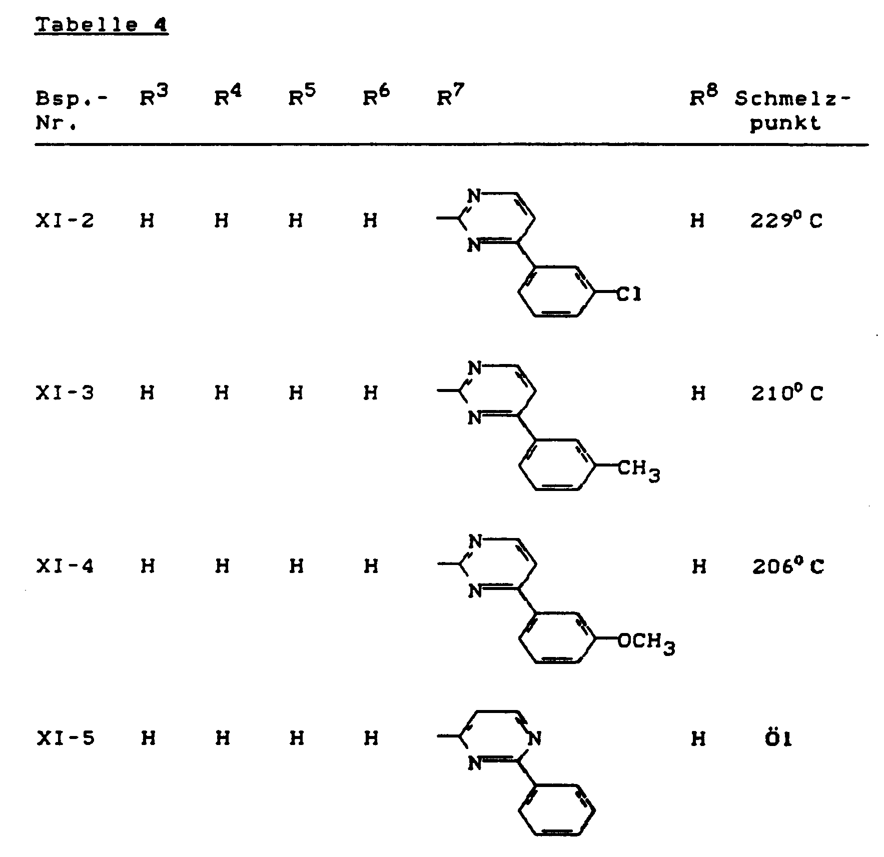

### Beispiel (XIII-1)

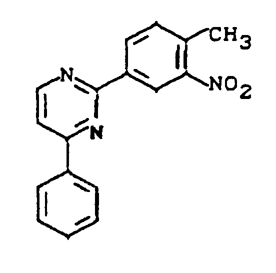

Man erhitzt ein Gemisch aus 24,0 g (111 mmol) 4-Methyl-3-nitro-benzamidin-Hydrochlorid, 17,5 g (97 mmol) 3-Dimethylamino-acryloylbenzol und 7,55 g (200 mmol) Natriummethanolat in 150 ml Ethanol 5 Stunden am Rückfluß zum Sieden. Nach dem Abkühlen wird das Reaktionsgemisch auf Eiswasser gegossen, der ausgefallene Feststoff abgesaugt und getrocknet.

Man erhält 20,0 g (79% der Theorie) an 2-Methyl-5-(4-phenylpyrimidin-2-yl)-nitrobenzol vom Schmelzpunkt 139°C.

Analog zum Herstellungsbeispiel (XIII-1) und unter Beachtung der allgemeinen Angaben zu den erfindungsgemäßen Verfahren können die in Tabelle 5 aufgeführten Verbindungen der Formel (XIII) erhalten werden.

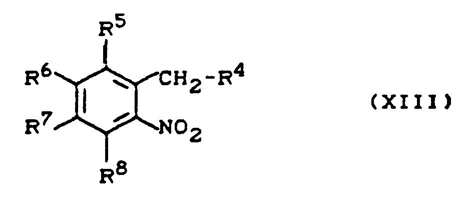

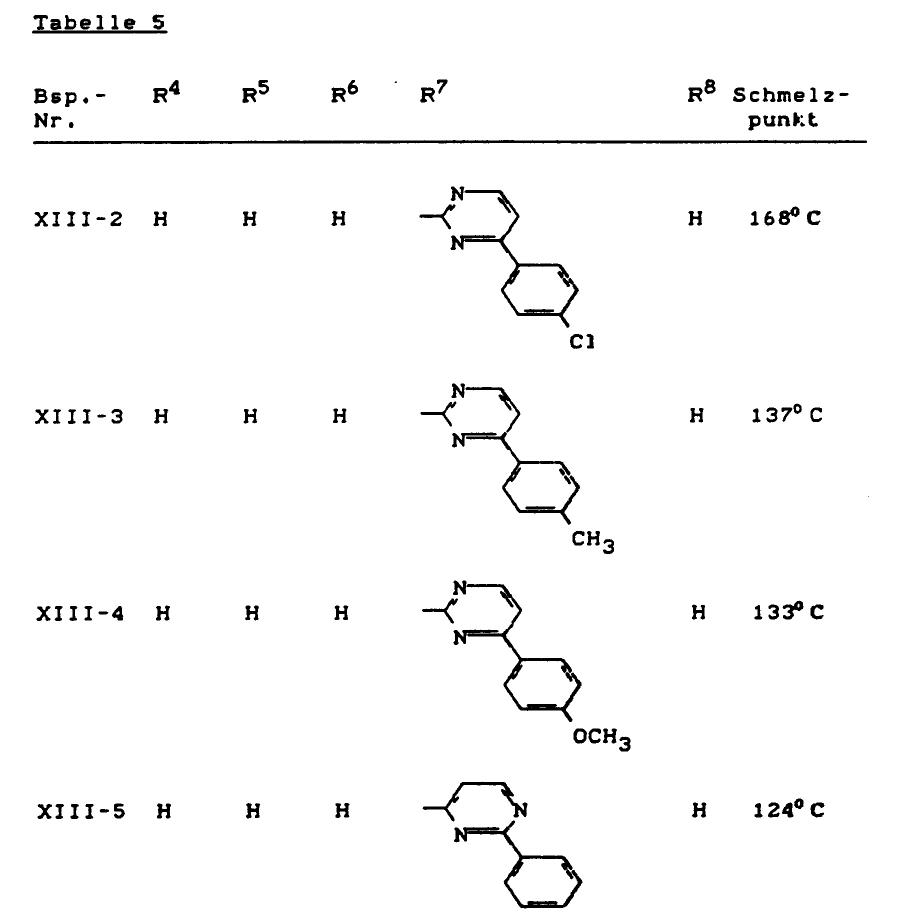

### Beispiel (XV-1)

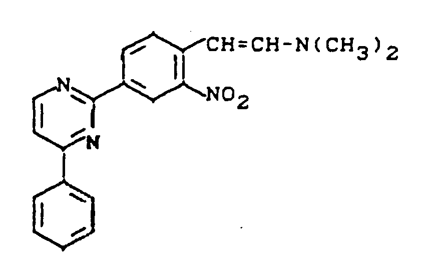

Eine Mischung aus 17,41 g (60 mmol) 2-Methyl-5-[4-(phenyl)-pyrimidin-2-yl]-nitrobenzol, 52 ml Dimethylformamiddimethylacetal und 70 ml Dimethylformamid wird 12 bis 20 Stunden, bis zum vollständigen Umsatz (dünnschichtchromatographische Kontrolle), am Rückfluß zum Sieden erhitzt. Danach engt man unter stark vermindertem Druck ein. Der ölige oder kristalline, schwarzrote Rückstand wird ohne weitere Reinigung weiterverarbeitet.

Man erhält β-Dimethylamino-4-[4-(phenyl)-pyrimidin-2-yl]-2-nitrostyrol als Öl.

Analog zum Herstellungsbeispiel (XV-1) können die in Tabelle 6 aufgeführten Verbindungen der Formel (XV) erhalten werden.

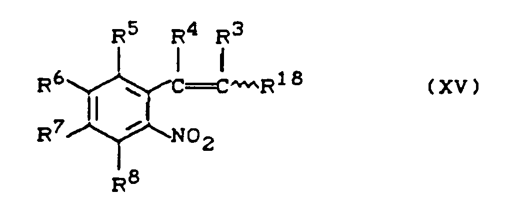

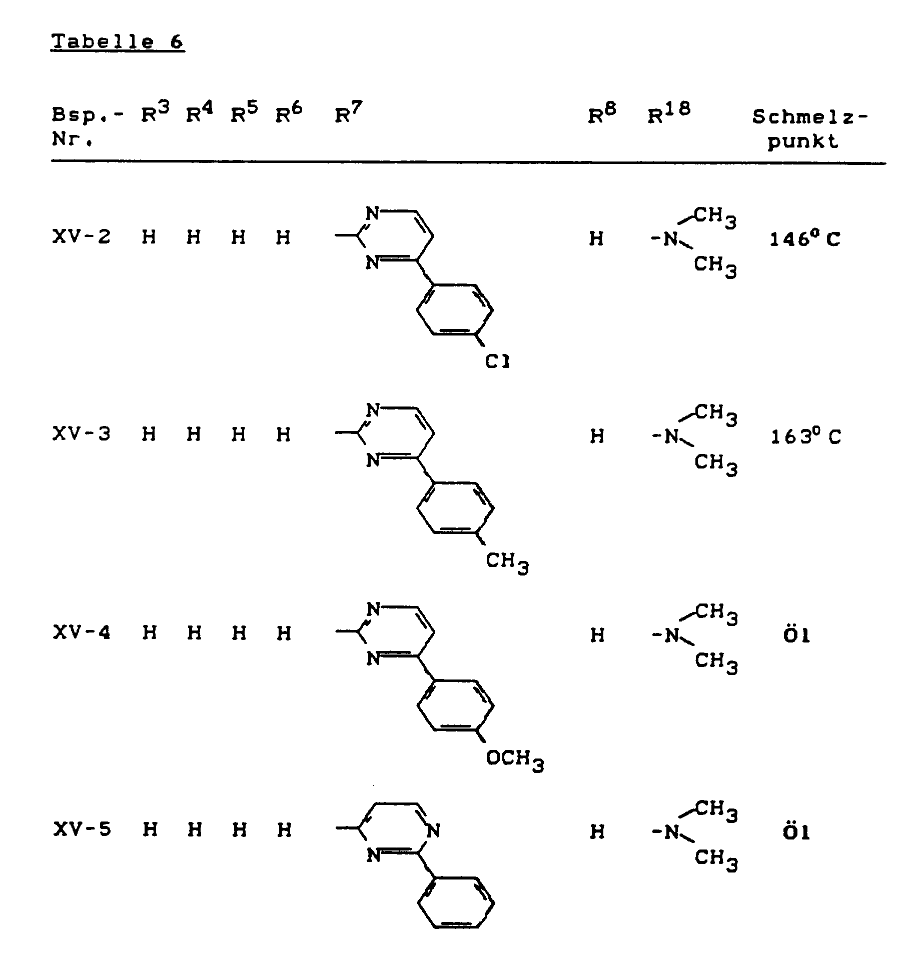

### Anwendungsbeispiele

In den folgenden Anwendungsbeispielen wurde die nachstehend aufgeführte Verbindung als Vergleichssubstanz eingesetzt: 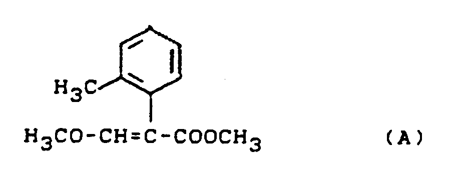
3-Methoxy-2-(2-methylphenyl)-acrylsäuremethylester (bekannt aus EP-A 178 816).

### Beispiel A

### Phaedon-Test

- Lösungsmittel:: 3 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlpflanzen (Brassica oleracea) werden mit der Wirkstoffzubereitung der gewünschten Konzentration behandelt. Ein Blatt der behandelten Pflanze wird in eine Plastikdose gelegt und mit Larven (L₃) des Meerrettichkäfers (Phaedon cochleariae) besetzt. Nach 2 bis 4 Tagen wird jeweils ein weiteres Blatt von derselben Pflanze für die Nachfütterung verwendet.

Nach der gewünschten Zeit wird die Abtötung in % bestimmt. Dabei bedeutet 100 %, daß alle Tiere abgetötet wurden; 0 % bedeutet, daß keine Tiere abgetötet wurden.

Bei diesem Test zeigt z.B. die folgende Verbindung der Herstellungsbeispiele überlegene Wirksamkeit gegenüber dem Stand der Technik: (1), (3), (4), (5) und (6).

### Beispiel B

### Plutella-Test

- Lösungsmittel:: 3 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlpflanzen (Brassica oleracea) werden mit der Wirkstoffzubereitung der gewünschten Konzentration behandelt. Ein Blatt der behandelten Pflanze wird in eine Plastikdose gelegt und mit Larven (L₂) der Kohlschabe (Plutella xylostella) besetzt. Nach 2 und 4 Tagen wird jeweils ein weiteres Blatt von derselben Pflanze für die Nachfütterung verwendet.

Nach der gewünschten Zeit wird die Abtötung in % bestimmt. Dabei bedeutet 100%, daß alle Tiere abgetötet wurden; 0% bedeutet, daß keine Tiere abgetötet wurden.

Bei diesem Test zeigt z.B. die folgende Verbindung der Herstellungsbeispiele überlegene Wirksamkeit gegenüber dem Stand der Technik: (1), (3), (4), (5) und (6).

### Beispiel C

### Spodoptera-Test

- Lösungsmittel:: 3 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojabohnenpflanzen (Glycine soja) werden mit der Wirkstoffzubereitung der gewünschten Konzentration behandelt. In zehnfacher Wiederholung wird je ein Blatt der behandelten Pflanze in eine Plastikdose gelegt und mit je einer Larve (L₂) des Heerwurms (Spodoptera frugiperda) besetzt. Nach 3 Tagen füttert man je Dose mit einem weiteren Blatt der entsprechenden Pflanze nach. Am 7.Tag werden die Larven auf unbehandeltes Kunstfutter umgesetzt.

Nach der gewünschten Zeit wird die Abtötung in % bestimmt. Dabei bedeutet 100%, daß alle Tiere abgetötet wurden; 0% bedeutet, daß keine Tiere abgetötet wurden.

Bei diesem Test zeigt z.B. die folgende Verbindung der Herstellungsbeispiele überlegene Wirksamkeit gegenüber dem Stand der Technik: (3) und (5).

### Beispiel D

### Nephotettix-Test

- Lösungsmittel:: 3 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichstteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigt z.B. die folgende Verbindung der Herstellungsbeispiele überlegene Wirksamkeit gegenüber dem Stand der Technik: (3) und (5).

### Beispiel E

### Myzus-Test

- Lösungsmittel:: 3 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der gewünschten Konzentration der Wirkstoffzubereitung werden Dicke Bohnen-Pflanzen (Vicia faba), welche mit der Grünen Pfirsichblattlaus (Myzus persicae) befallen sind, durch Tauchen behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigt z.B. die folgende Verbindung der Herstellungsbeispiele überlegene Wirksamkeit gegenüber dem Stand der Technik: (1), (3), (4) und (5).

### Beispiel F

### Tetranychus-Test (OP-resistent)

- Lösungsmittel:: 3 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigt z.B. die folgende Verbindung der Herstellungsbeispiele überlegene Wirksamkeit gegenüber dem Stand der Technik: (3) und (5).

### Beispiel G

### Ovizide Wirkung auf Eigelege von Heliothis armigera (Baumwollkapselwurm)

- Lösungsmittel:: 3 Gewichtsteile Dimethylformamid
- Emulgator :: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

In die Wirkstoffzubereitungen der gewünschten Konzentration wurden 2 Tage alte Eigelege auf Filterpapier für 30 Sekunden eingetaucht und für 6 Tage im Labor unter Langtagbedingungen in geschlossenen Petrischalen deponiert. Kriterium für die Wirkungsbeurteilung war die prozentuale Schlupfverhinderung im Vergleich zu unbehandelten Eigelegen.

Bei diesem Test zeigt z.B. die folgende Verbindung der Herstellungsbeispiele überlegene Wirksamkeit gegenüber dem Stand der Technik: (1), (3) und (4).

### Beispiel H

### Pyricularia-Test (Reis)/protektiv

- Lösungsmittel:: 12,5 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100% relativer Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigt in diesem Test z.B. die Verbindung des Herstellungsbeispiels (6).

## Patentansprüche

1. Substituierte 2-[6-(Pyrimidinyl)-indol-1-yl]-acrylsäureester der allgemeinen Formel (I) 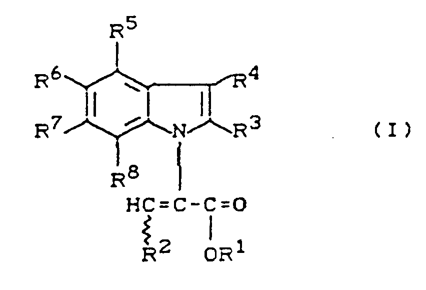 in welcher
R¹ für geradkettiges oder verzweigtes Alkyl mit 1 bis 6 Kohlenstoffatomen oder für Benzyl steht,
R² für Dialkylamino mit jeweils 1 bis 6 Kohlenstoffatomen in den einzelnen geradkettigen oder verzweigten Alkylteilen, jeweils geradkettiges oder verzweigtes Alkoxy oder Alkylthio mit 1 bis 6 Kohlenstoffatomen oder für jeweils gegebenenfalls einfach oder mehrfach, gleich oder verschieden substituiertes Benzyloxy oder Benzylthio steht, wobei als Phenylsubstituenten infrage kommen:
Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen oder Cycloalkyl mit 3 bis 7 Kohlenstoffatomen,
R³ und R⁴ jeweils unabhängig voneinander für Wasserstoff, Cyano, Fluor, Chlor, Brom oder geradkettiges oder verzweigtes Alkyl mit 1 bis 6 Kohlenstoffatomen stehen,
R⁵, R⁶ und R⁸ unabhängig voneinander jeweils für Wasserstoff, Fluor, Chlor, Brom, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Cycloalkyl mit 3 bis 7 Kohlenstoffatomen, jeweils unsubstituiertes oder im Arylteil einfach bis dreifach, gleich oder verschieden durch Fluor, Chlor, Brom, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Phenyl, Benzyl, Phenoxy, Phenylthio, Benzyloxy oder Benzylthio oder jeweils gegebenenfalls im Heteroarylteil einfach oder zweifach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Pyridyl, Thienyl, Furyl, Pyridazinyl, Pyrazinyl, Thiazolyl, Pyridylmethyl, Thienylmethyl, Furylmethyl, Pyridyloxy, Thienyloxy, Thiazolyloxy, Pyridylmethyloxy, Thienylmethyloxy, Thienylthio, Furylthio, Pyridylmethylthio oder Thienylmethylthio stehen, oder
R⁵ und R⁶ gemeinsam für Alkandiyl mit 3 bis 5 Kohlenstoffatomen oder für Alkylidendioxy mit 1 bis 3 Kohlenstoffatomen stehen und
R⁷ für eine der folgenden Gruppierungen steht 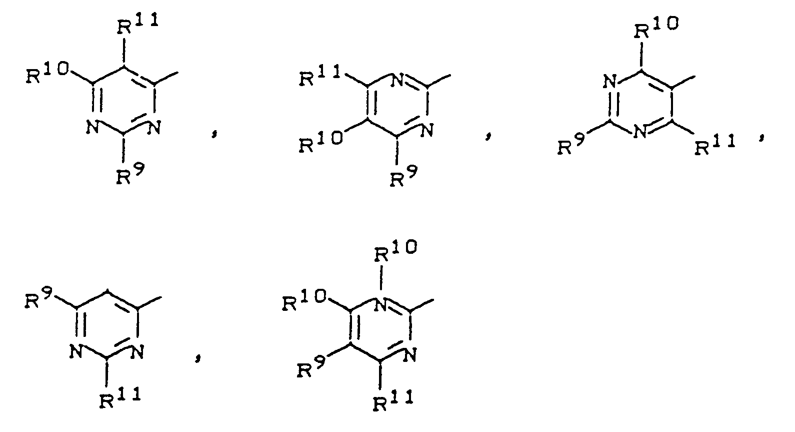 wobei
R⁹, R¹⁰ und R¹¹ jeweils unabhängig voneinander für Wasserstoff, Fluor, Chlor, Brom, Cyano, jeweils geradkettiges oder verzweigtes Alkyl, Alkoxy, Alkylthio oder Halogenalkyl mit 1 bis 6 Kohlenstoffatomen und gegebenenfalls 1 bis 13 gleichen oder verschiedenen Halogenatomen steht, für Cycloalkyl mit 3 bis 7 Kohlenstoffatomen, für geradkettiges oder verzweigtes Alkoxycarbonyl mit 1 bis 6 Kohlenstoffatomen, für jeweils in den einzelnen Alkylteilen geradkettiges oder verzweigtes, gegebenenfalls gleich oder verschieden substituiertes Dialkylaminocarbonyl, Alkylaminocarbonyl, Dialkylamino oder Alkylamino mit jeweils 1 bis 4 Kohlenstoffatomen oder für jeweils unsubstituiertes oder einfach bis dreifach, gleich oder verschieden substituiertes Phenyl, Benzyl, Phenyloxy, Phenylthio, Benzyloxy oder Benzylthio steht oder für unsubstituiertes oder einfach oder zweifach, gleich oder verschieden substituiertes Pyrrolyl, Thienyl, Furyl, Thiazolyl, Isothiazolyl, Oxazolyl, Isoxazolyl, Pyrazolyl, Imidazolyl, Pyridyl, Pyrimidyl, Pyridazinyl, Pyrazinyl, Triazinyl, Pyrrolyloxy, Thienyloxy, Furyloxy, Thiazolyloxy, Isothiazolyloxy, Oxazolyloxy, Isoxazolyloxy, Pyrazolyloxy, Imidazolyloxy, Pyridyloxy, Pyrimidyloxy, Pyridazinyloxy, Pyrazinyloxy, Pyrrolylthio, Thienylthio, Furylthio, Thiazolythio, Isothiazolylthio, Oxazolythio, Isoxazolylthio, Pyrazolylthio, Imidazolylthio, Pyridylthio, Pyrimidylthio, Pyridazinylthio oder Pyrazinylthio steht, wobei als Substituenten jeweils infrage kommen: Halogen, jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und jeweils gegebenenfalls 1 bis 9 gleichen oder verschiedenen Halogenatomen, Alkylcarbonylamino mit 1 bis 4 Kohlenstoffatomen im geradkettigen oder verzweigten Alkylteil, Dialkylamino oder Dialkylaminocarbonyl mit jeweils 1 bis 4 Kohlenstoffatomen in den jeweiligen geradkettigen oder verzweigten Alkylteilen sowie jeweils gegebenenfalls substituiertes Phenyl oder Benzyl, oder
zwei benachbarte Substituenten R⁹ und R¹⁰, bzw. R¹⁰ und R¹¹ oder R⁹ und R¹¹ gemeinsam für Alkandiyl mit 3 oder 4 Kohlenstoffatomen stehen.

2. Substituierte 2-[6-(Pyrimidinyl)-indol-1-yl]-acrylsäureester der allgemeinen Formel (I) gemäß Anspruch 1, in welcher
R¹ für geradkettiges oder verzweigtes Alkyl mit 1 bis 4 Kohlenstoffatomen steht,
R² für Dialkylamino mit jeweils 1 bis 4 Kohlenstoffatomen in den jeweiligen geradkettigen oder verzweigten Alkylteilen, jeweils geradkettiges oder verzweigtes Alkoxy oder Alkylthio mit 1 bis 4 Kohlenstoffatomen oder für Benzyloxy oder Benzylthio steht,
R³ und R⁴ gleich oder verschieden sind und für Wasserstoff, Cyano, Fluor, Chlor, Brom, Methyl, Ethyl, n- oder i-Propyl, n-, i-, s- oder t-Butyl stehen,
R⁵ und R⁶ gleich oder verschieden sind und für Wasserstoff, Fluor, Chlor, Brom, Cyano, Nitro, Methyl, Ethyl, n- oder i-Propyl, n-, i-, s-oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl oder Cyclohexyl stehen, oder
R⁵ und R⁶ gemeinsam für eine Methylendioxy-, 1,3-Propandiyl- oder 1,4-Butandiylgruppierung stehen,
R⁷ für eine der folgenden Gruppierungen steht, 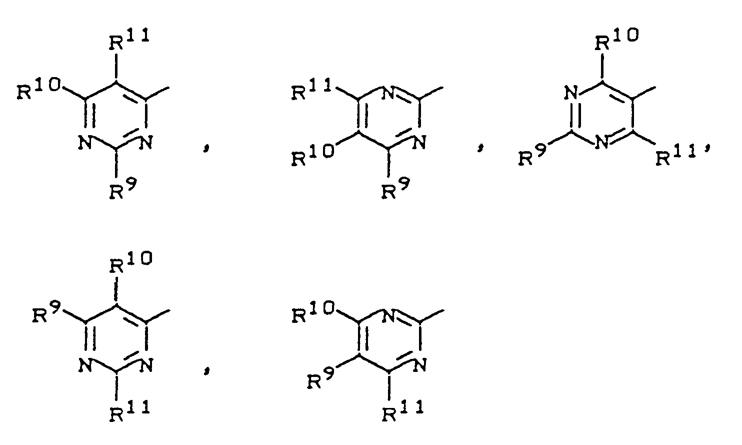 wobei
R⁹ für Wasserstoff, jeweils geradkettiges oder verzweigtes Alkyl, Alkoxy oder Alkylthio mit jeweils 1 bis 6 Kohlenstoffatomen, Cyclopentyl, Cyclohexyl, Methylamino, Ethylamino, Dimethylamino, Diethylamino, jeweils gegebenenfalls einfach bis dreifach, gleich oder verschieden substituiertes Phenyl, Benzyl, Phenoxy, Phenylthio, Benzyloxy oder Benzylthio oder für gegebenenfalls ein- oder zweifach gleich oder verschieden substituiertes Pyrrolyl, Thienyl, Furyl, Thiazolyl, Isothiazolyl, Oxazolyl, Isoxazolyl, Pyrazolyl, Imidazolyl, Triazinyl, Pyridyl, Pyrimidyl, Pyridazinyl, Pyrazinyl, Pyridylmethyl, Thienylmethyl, Furylmethyl, Pyridyloxy, Thienyloxy, Thiazolyloxy, Pyridylmethyloxy, Thienylmethyloxy, Thienylthio, Furylthio, Pyridylmethylthio oder Thienylmethylthio steht, wobei als Substituenten jeweils Halogen, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n-oder i-Propylthio, n-, i-, s- oder t-Butylthio, Methylcarbonylamino, Ethylcarbonylamino, Methylcarbonyl, Ethylcarbonyl, n- oder i-Propylcarbonyl, n-, i-, s- oder t-Butylcarbonyl, Halogenalkyl, Halogenalkoxy oder Halogenalkylthio mit jeweils 1 oder 2 Kohlenstoffatomen und 1 bis 5 gleichen oder verschiedenen Fluor- oder Chloratomen, Dialkylamino oder Dialkylaminocarbonyl mit 1 oder 2 Kohlenstoffatomen in den einzelnen Alkylteilen, unsubstituiertes oder einfach bis zweifach, gleich oder verschieden substituiertes Phenyl oder Benzyl genannt seien, wobei als Phenyl- bzw. Benzylsubstituenten jeweils Fluor, Chlor, Methyl, Methoxy oder Phenoxy in Frage kommen und
R¹⁰ und R¹¹ gleich oder verschieden sind und für Wasserstoff, geradkettiges oder verzweigtes Alkyl mit 1 bis 4 Kohlenstoffatomen, Chlor, Brom, Cyano, Methoxycarbonyl, Ethoxycarbonyl, Dimethylaminocarbonyl, Diethylaminocarbonyl, Methylaminocarbonyl, Ethylaminocarbonyl, Methoxy, Ethoxy, Methylthio oder Ethylthio steht oder
R¹⁰ und R¹¹ gemeinsam für eine Methylendioxy-, 1,3-Propandiyl- oder 1,4-Butandiylgruppierung stehen und
R⁸ für Wasserstoff, Fluor, Chlor, Brom, Methyl, Ethyl, n- oder i-Propyl, Methoxy, Ethoxy, n-oder i-Propoxy, Methylthio, Trifluormethyl, Trifluormethoxy, Difluormethoxy, Trifluormethylthio, Methoxycarbonyl, Ethoxycarbonyl, Methoximininomethyl oder Ethoximinomethyl steht.

3. Verfahren zur Herstellung von substituierten 2-[6-(Pyrimidinyl)-indol-1-yl]-acrylsäureestern der allgemeinen Formel (I) 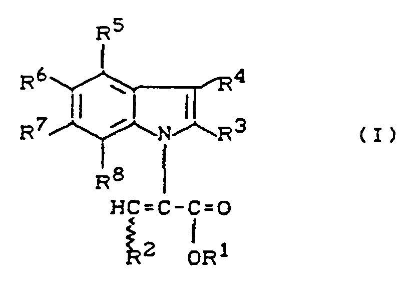 in welcher
R¹, R², R³, R⁴, R⁵, R⁶, R⁷ und R⁸ die in Anspruch 1 angegebene Bedeutung haben,
dadurch gekennzeichnet, daß man
a) zum Erhalt von substituierten 2-[6-(Pyrimidinyl)-indol-1-yl]-acrylsäureestern der allgemeinen Formel (Ia) 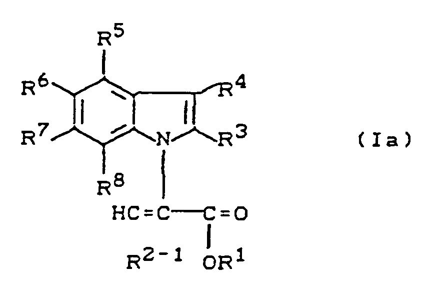 in welcher
R¹, R³, R⁴, R⁵, R⁶, R⁷ und R⁸ die oben angegebene Bedeutung haben und
R²⁻¹ für Alkoxy oder unsubstituiertes oder substituiertes Aralkyloxy steht,
Hydroxyacrylsäureester oder deren Alkalimetallsalze der Formel (II) 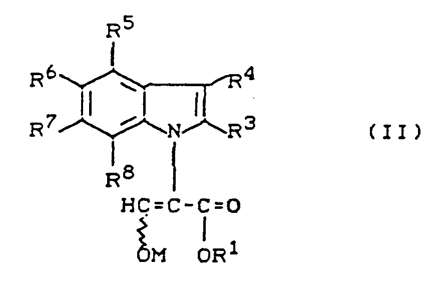 in welcher
M für Wasserstoff oder für ein Alkalimetallkation steht und
R¹, R³, R⁴, R⁵, R⁶, R⁷ und R⁸ die oben angegebene Bedeutung haben,
mit Alkylierungsmitteln der Formel (III)
R¹²-E¹ (III)
in welcher
R¹² für Alkyl oder unsubstituiertes oder substituiertes Aralkyl steht und
E¹ für eine elektronenanziehende Abgangsgruppe steht,
gegebenenfalls in Gegenwart eines Verdünnungsmittels und gegebenenfalls in Gegenwart eines Reaktionshilfsmittels umsetzt;
b) oder daß man zum Erhalt von substituierten 2-[6-(Pyrimidinyl)-indol-1-yl]-acrylsäureestern der allgemeinen Formel (Ib) 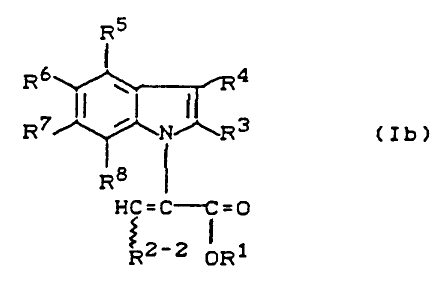 in welcher
R¹, R³, R⁴, R⁵, R⁶, R⁷ und R⁸ die oben angegebene Bedeutung haben und
R²⁻² für Dialkylamino steht,
substituierte Essigsäureester der Formel (IV) 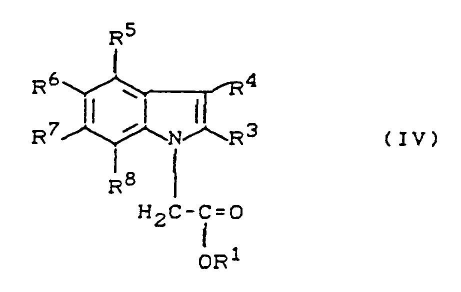 in welcher
R¹, R³, R⁴, R⁵, R⁶, R⁷ und R⁸ die oben angegebene Bedeutung haben,
mit Formamiden der Formel (Va) 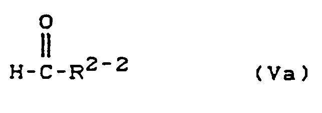 in welcher
R²⁻² die oben angegebene Bedeutung hat,
oder mit Formamid-Derivaten der Formel (Vb) 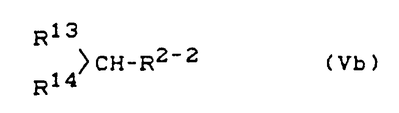 in welcher
R¹³ und R¹⁴ unabhängig voneinander für Alkoxy oder Dialkylamino stehen und
R²⁻² die oben angegebene Bedeutung hat,
gegebenenfalls in Gegenwart eines Verdünnungsmittels umsetzt;
c) oder daß man zum Erhalt von substituierten 2-[6-(Pyrimidinyl)-indol-1-yl]-acrylsäureestern der Formel (Ic) 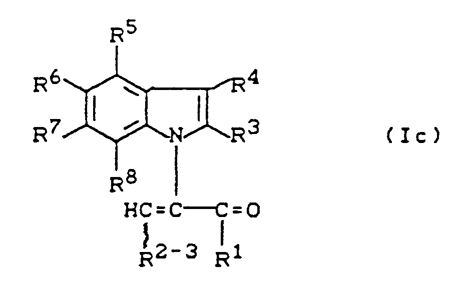 in welcher
R¹, R³, R⁴, R⁵, R⁶, R⁷ und R⁸ die oben angegebene Bedeutung haben und
R²⁻³ für Alkylthio oder unsubstituiertes oder substituiertes Aralkylthio steht,
Ketocarbonsäurederivate der Formel (VI) 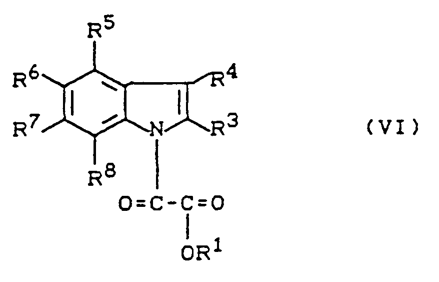 in welcher
R¹, R³, R⁴, R⁵, R⁶, R⁷ und R⁸ die oben angegebene Bedeutung haben,
mit metallorganischen Verbindungen der Formel (VII) 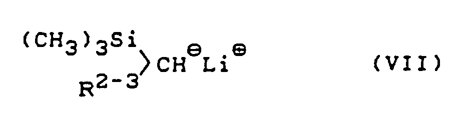 in welcher
R²⁻³ die oben angegebene Bedeutung hat,
gegebenenfalls in Gegenwart eines Verdünnungsmittels umsetzt;
d) oder daß man zum Erhalt von substituierten 2-[6-(Pyrimidinyl)-indol-1-yl]-acrylsäureestern der Formel (Ic) weiterhin,
substituierte Acrylsäureester der Formel (VIII) 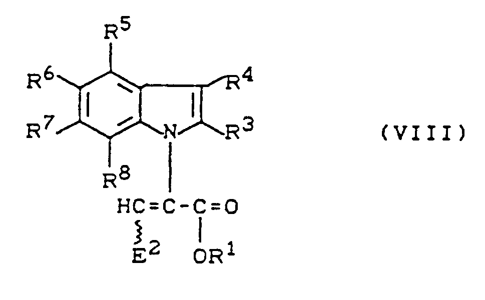 in welcher
R¹, R³, R⁴, R⁵, R⁶, R⁷ und R⁸ die oben angegebene Bedeutung haben und
E² für eine elektronenanziehende Abgangsgruppe steht,
mit Thiolen der Formel (IX)
R²⁻³-H (IX)
in welcher
R²⁻³ die oben angegebene Bedeutung hat,
gegebenenfalls in Gegenwart eines Verdünnungsmittels und gegebenenfalls in Gegenwart eines Reaktionshilfsmittels umsetzt.

4. Hydroxyacrylsäureester-Derivate der allgemeinen Formel 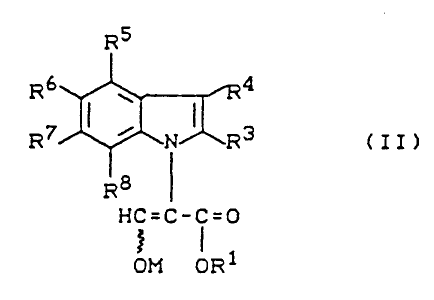 in welcher
M für Wasserstoff oder für ein Alkalimetallkation steht und
R¹, R³, R⁴, R⁵, R⁶, R⁷ und R⁸ die in Anspruch 1 angegebene Bedeutung besitzen.

5. Verfahren zur Herstellung von Hydroxyacrylsäureester-Derivaten der allgemeinen Formel (II) 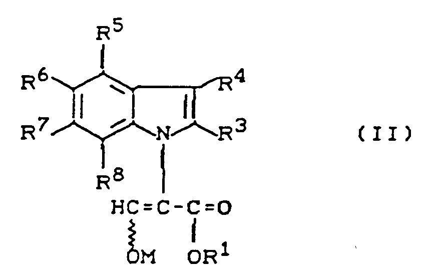 in welcher
M für Wasserstoff oder für ein Alkalimetallkation steht und
R¹, R³, R⁴, R⁵, R⁶, R⁷ und R⁸ die in Anspruch 1 angegebene Bedeutung besitzen,
dadurch gekennzeichnet, daß man substituierte Essigsäureester-Derivate der Formel (IV) 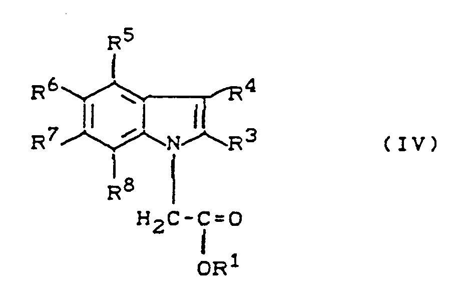 in welcher
R¹, R³, R⁴, R⁵, R⁶, R⁷ und R⁸ die oben angegebene Bedeutung haben,
mit Ameisensäureestern der Formel (X) 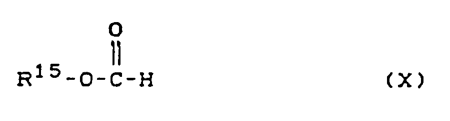 in welcher
R¹⁵ für Alkyl steht,
gegebenenfalls in Gegenwart eines Verdünnungsmittels und gegebenenfalls in Gegenwart eines basischen Reaktionshilfsmittels bei Temperaturen von -20°C bis +50°C umsetzt.

6. Substituiertes Essigsäureester-Derivate der allgemeinen Formel (IV) 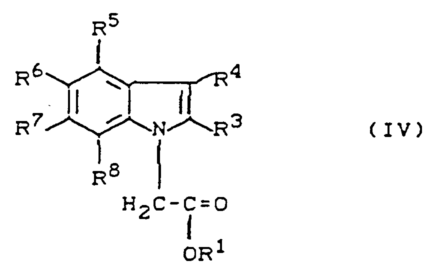 in welcher
R¹, R³, R⁴, R⁵, R⁶, R⁷ und R⁸ die Anspruch 1 angegebene Bedeutung besitzen.

7. Verfahren zur Herstellung von Essigsäureester-Derivaten der allgemeinen Formel (IV) 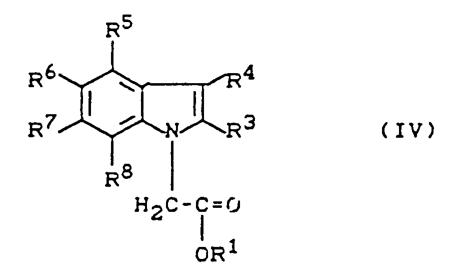 in welcher
R¹, R³, R⁴, R⁵, R⁶, R⁷ und R⁸ die in Anspruch 1 angegebene Bedeutung besitzen,
dadurch gekennzeichnet, daß man Indolderivate der allgemeinen Formel (XI) 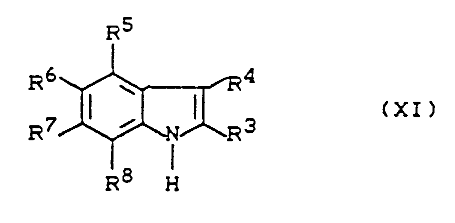 in welcher
R³, R⁴, R⁵, R⁶, R⁷ und R⁸ die oben angegebene Bedeutung besitzen,
mit Essigsäurederivaten der allgemeinen Formel (XII)
E³-CH₂-COOR¹ (XII)
in welcher
R¹ die oben angegebene Bedeutung hat und
E³ für eine elektronenanziehende Abgangsgruppe steht,
gegebenenfalls in Gegenwart eines Verdünnungsmittels und gegebenenfalls in Gegenwart eines basischen Hilfsmittels bei Temperaturen zwischen -20°C und +100°C umsetzt.

8. Schädlingsbekämpfungsmittel, gekennzeichnet durch einen Gehalt an mindestens einem substituierten 2-[6-(Pyrimidinyl)-indol-1-yl]-acrylsäureester der Formel (I) nach Anspruch 1.

9. Verfahren zur Bekämpfung von landwirtschaftlichen Schädlingen, dadurch gekennzeichnet, daß man substituierte 2-[6-(Pyrimidinyl)-indol-1-yl]-acrylsäureester der Formel (I) nach Anspruch 1 auf landwirtschaftliche Schädlinge und/oder ihren Lebensraum einwirken läßt.

10. Verwendung von substituierten 2-[6-Pyrimidinyl)-indol-1-yl)-acrylsäureester der Formel (I) nach Anspruch 1 zur Bekämpfung von landwirtschaftlichen Schädlingen.

11. Verfahren zur Herstellung von Schädlingsbekämpfungsmitteln, dadurch gekennzeichnet, daß man substituierte 2-[6-(Pyrimidinyl)-indol-1-yl]-acrylsäureester der Formel (I) nach Anspruch 1 mit Streckmitteln und/oder oberflächenaktiven Mitteln vermischt.

12. Schädlingsbekämpfungsmittel, gekennzeichnet durch einen Gehalt an mindestens einem substituierten 2-[6-(Pyrimidinyl)-indol-1-yl]-acrylsäureester der Formel (IV) nach Anspruch 6.

13. Verfahren zur Bekämpfung von landwirtschaftlichen Schädlingen, dadurch gekennzeichnet, daß man substituierte 2-[6-(Pyrimidinyl)-indol-1-yl]-acrylsäureester der Formel (IV) nach Anspruch 6 auf landwirtschaftliche Schädlinge und/oder ihren Lebensraum einwirken läßt.

14. Verwendung von substituierten 2-[6-Pyrimidinyl)-indol-1-yl)-acrylsäureester der Formel (IV) nach Anspruch 6 zur Bekämpfung von landwirtschaftlichen Schädlingen.

15. Verfahren zur Herstellung von Schädlingsbekämpfungsmitteln, dadurch gekennzeichnet, daß man substituierte 2-[6-Pyrimidinyl)-indol-1-yl)-acrylsäureester der Formel (IV) nach Anspruch 6 mit Streckmitteln und/oder oberflächenaktiven Mitteln vermischt.

## Claims

1. Substituted 2-[6-(pyrimidinyl)-indol-1-yl]-acrylic esters of the general formula (I) 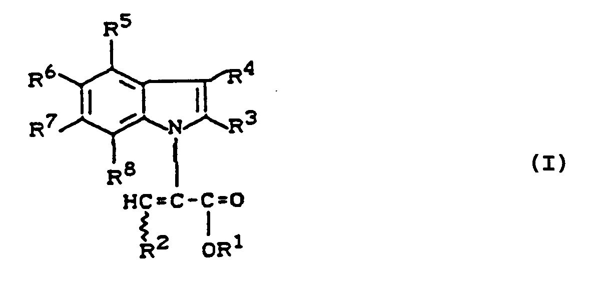 in which
R¹ represents straight-chain or branched alkyl having 1 to 6 carbon atoms, or represents benzyl,
R² represents dialkylamino having in each case 1 to 6 carbon atoms in the individual straight-chain or branched alkyl moieties, in each case straight-chain or branched alkoxy or alkylthio having 1 to 6 carbon atoms, or represents benzyloxy or benzylthio, each of which is optionally monosubstituted or polysubstituted by identical or different substituents, suitable phenyl substituents being:
halogen, in each case straight-chain or branched alkyl, alkoxy or alkylthio each having 1 to 4 carbon atoms, in each case straight-chain or branched halogenoalkyl, halogenoalkoxy or halogenoalkylthio each having 1 to 4 carbon atoms and 1 to 9 identical or different halogen atoms, or cycloalkyl having 3 to 7 carbon atoms,
R³ and R⁴ in each case independently of one another represent hydrogen, cyano, fluorine, chlorine, bromine or straight-chain or branched alkyl having 1 to 6 carbon atoms,
R⁵, R⁶ and R⁸ independently of one another in each case represent hydrogen, fluorine, chlorine, bromine, cyano, nitro, in each case straight-chain or branched alkyl, alkoxy or alkylthio, each of which has 1 to 4 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 4 carbon atoms in the individual alkyl moieties, cycloalkyl having 3 to 7 carbon atoms, or represent phenyl, benzyl, phenoxy, phenylthio, benzyloxy or benzylthio, each of which is unsubstituted or monosubstituted to trisubstituted in the aryl moiety by identical or different substituents from the series comprising fluorine, chlorine, bromine, alkyl, alkoxy, alkylthio, halogenoalkyl, halogenoalkoxy or halogenoalkylthio, each of which has 1 to 4 carbon atoms and if appropriate 1 to 9 identical or different halogen atoms, or represent pyridyl, thienyl, furyl, pyridazinyl, pyrazinyl, thiazolyl, pyridylmethyl, thienylmethyl, furylmethyl, pyridyloxy, thienyloxy, thiazolyloxy, pyridylmethyloxy, thienylmethyloxy, thienylthio, furylthio, pyridylmethylthio or thienylmethylthio, each of which is optionally monosubstituted or disubstituted in the heteroaryl moiety by identical or different substituents from the series comprising halogen, alkyl, alkoxy, alkylthio, halogenoalkyl, halogenoalkoxy or halogenoalkylthio, each of which has 1 to 4 carbon atoms and if appropriate 1 to 9 identical or different halogen atoms, or
R⁵ and R⁶ together represent alkanediyl having 3 to 5 carbon atoms, or represent alkylidenedioxy having 1 to 3 carbon atoms, and
R⁷ represents one of the following groups 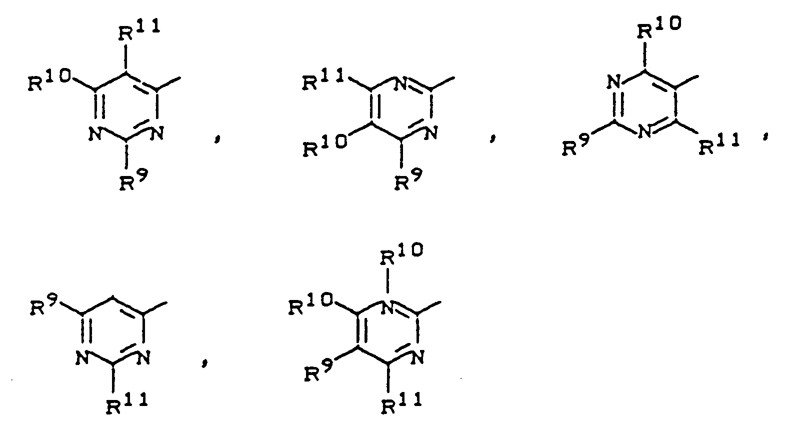 where
R⁹, R¹⁰ and R¹¹ in each case independently of one another represent hydrogen, fluorine, chlorine, bromine, cyano, in each case straight-chain or branched alkyl, alkoxy, alkylthio or halogenoalkyl having 1 to 6 carbon atoms and if appropriate 1 to 13 identical or different halogen atoms, or represent cycloalkyl having 3 to 7 carbon atoms, or represent straight-chain or branched alkoxycarbonyl having 1 to 6 carbon atoms, or represent dialkylaminocarbonyl, alkylaminocarbonyl, dialkylamino or alkylamino, each of which has 1 to 4 carbon atoms, each of which is straight-chain or branched in the individual alkyl moieties and each of which is optionally substituted by identical or different substituents, or represents phenyl, benzyl, phenyloxy, phenylthio, benzyloxy or benzylthio, each of which is unsubstituted or monosubstituted to trisubstituted by identical or different substituents, or represents pyrrolyl, thienyl, furyl, thiazolyl, isothiazolyl, oxazolyl, isoxazolyl, pyrazolyl, imidazolyl, pyridyl, pyrimidyl, pyridazinyl, pyrazinyl, triazinyl, pyrrolyloxy, thienyloxy, furyloxy, thiazolyloxy, isothiazolyloxy, oxazolyloxy, isoxazolyloxy, pyrazolyloxy, imidazolyloxy, pyridyloxy, pyrimidyloxy, pyridazinyloxy, pyrazinyloxy, pyrrolylthio, thienylthio, furylthio, thiazolylthio, isothiazolylthio, oxazolylthio, isoxazolylthio, pyrazolylthio, imidazolylthio, pyridylthio, pyrimidylthio, pyridazinylthio or pyrazinylthio, each of which is unsubstituted or monosubstituted or disubstituted by identical or different substituents, suitable substituents in each case being: halogen, in each case straight-chain or branched alkyl, alkoxy, alkylthio, halogenoalkyl, halogenoalkoxy or halogenoalkylthio, each of which has 1 to 4 carbon atoms and, if appropriate, in each case 1 to 9 identical or different halogen atoms, alkylcarbonylamino having 1 to 4 carbon atoms in the straight-chain or branched alkyl moiety, dialkylamino or dialkylaminocarbonyl, each of which has 1 to 4 carbon atoms in the respective straight-chain or branched alkyl moieties, and in each case optionally substituted phenyl or benzyl, or
two adjacent substituents R⁹ and R¹⁰, or R¹⁰ and R¹¹, or R⁹ and R¹¹, together represent alkanediyl having 3 or 4 carbon atoms.

2. Substituted 2-[6-(pyrimidinyl)-indol-1-yl]-acrylic esters of the general formula (I) according to Claim 1, in which
R¹ represents straight-chain or branched alkyl having 1 to 4 carbon atoms,
R² represents dialkylamino having in each case 1 to 4 carbon atoms in the respective straight-chain or branched alkyl moieties, or represents in each case straight-chain or branched alkoxy or alkylthio having 1 to 4 carbon atoms, or represents benzyloxy or benzylthio,
R³ and R⁴ are identical or different and represent hydrogen, cyano, fluorine, chlorine, bromine, methyl, ethyl, n- or i-propyl or n-, i-, s- or t-butyl,
R⁵ and R⁶ are identical or different and represent hydrogen, fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy, difluoromethoxy, trifluoromethylthio, methoxycarbonyl, ethoxycarbonyl, methoximinomethyl, ethoximinomethyl, methoximinoethyl, ethoximinoethyl, cyclopentyl or cyclohexyl, or
R⁵ and R⁶ together represent a methylenedioxy, 1,3-propanediyl or 1,4-butanediyl group,
R⁷ represents one of the following groups 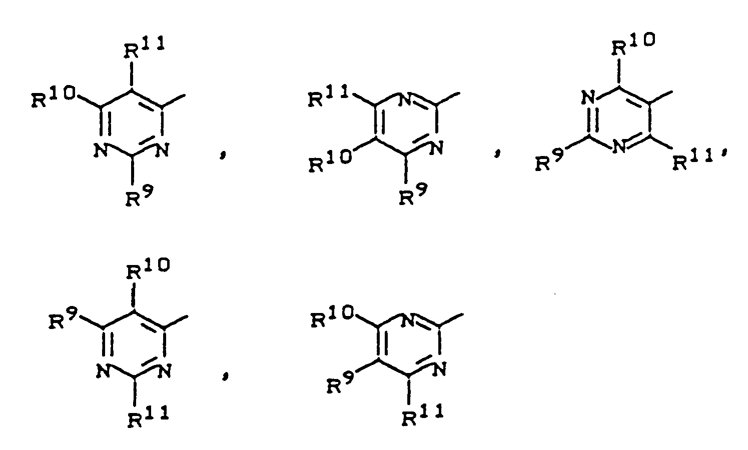 where
R⁹ represents hydrogen, in each case straight-chain or branched alkyl, alkoxy or alkylthio, each of which has 1 to 6 carbon atoms, cyclopentyl, cyclohexyl, methylamino, ethylamino, dimethylamino, diethylamino, or represents phenyl, benzyl, phenoxy, phenylthio, benzyloxy or benzylthio, each of which is optionally monosubstituted to trisubstituted by identical or different substituents, or represents pyrrolyl, thienyl, furyl, thiazolyl, isothiazolyl, oxazolyl, isoxazolyl, pyrazolyl, imidazolyl, triazinyl, pyridyl, pyrimidyl, pyridazinyl, pyrazinyl, pyridylmethyl, thienylmethyl, furylmethyl, pyridyloxy, thienyloxy, thiazolyloxy, pyridylmethyloxy, thienylmethyloxy, thienylthio, furylthio, pyridylmethylthio or thienylmethylthio, each of which is optionally monosubstituted or disubstituted by identical or different substituents, suitable substituents in each case being halogen, methyl, ethyl, n- or i-propyl, n-, i-, s-or t-butyl, methoxy, ethoxy, n- or i-propoxy, n-, i-, s- or t-butoxy, methylthio, ethylthio, n- or i-propylthio, n-, i-, s- or t-butylthio, methylcarbonylamino, ethylcarbonylamino, methylcarbonyl, ethylcarbonyl, n- or i-propylcarbonyl, n-, i-, s- or t-butylcarbonyl or halogenoalkyl, halogenoalkoxy or halogenoalkylthio, each of which has 1 or 2 carbon atoms and 1 to 5 identical or different fluorine or chlorine atoms, dialkylamino or dialkylaminocarbonyl having 1 or 2 carbon atoms in the individual alkyl moieties, or phenyl or benzyl, each of which are unsubstituted or monosubstituted to disubstituted by identical or different substituents, suitable phenyl or benzyl substituents in each case being fluorine, chlorine, methyl, methoxy or phenoxy, and
R¹⁰ and R¹¹ are identical or different and represent hydrogen, straight-chain or branched alkyl having 1 to 4 carbon atoms, chlorine, bromine, cyano, methoxycarbonyl, ethoxycarbonyl, dimethylaminocarbonyl, diethylaminocarbonyl, methylaminocarbonyl, ethylaminocarbonyl, methoxy, ethoxy, methylthio or ethylthio, or
R¹⁰ and R¹¹ together represent a methylenedioxy, 1,3-propanediyl or 1,4-butanediyl group, and
R⁸ represents hydrogen, fluorine, chlorine, bromine, methyl, ethyl, n- or i-propyl, methoxy, ethoxy, n- or i-propoxy, methylthio, trifluoromethyl, trifluoromethoxy, difluoromethoxy, trifluoromethylthio, methoxycarbonyl, ethoxycarbonyl, methoximinomethyl or ethoximinomethyl.

3. Process for the preparation of substituted 2-[6-(pyrimidinyl)-indol-1-yl]-acrylic esters of the general formula (I) 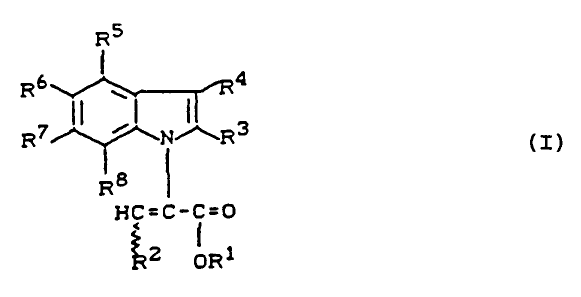 in which
R¹, R², R³, R⁴, R⁵, R⁶, R⁷ and R⁸ have the meaning mentioned in Claim 1,
characterized in that
a) to obtain substituted 2-[6-(pyrimidinyl)-indol-1-yl]-acrylic esters of the general formula (Ia) 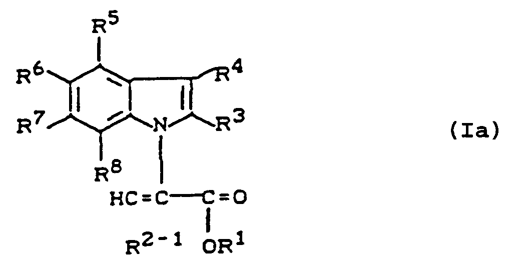 in which
R¹, R³, R⁴, R⁵, R⁶, R⁷ and R⁸ have the abovementioned meaning and
R²⁻¹ represents alkoxy or unsubstituted or substituted aralkyloxy,
hydroxyacrylic esters or alkali metal salts thereof of the formula (II) 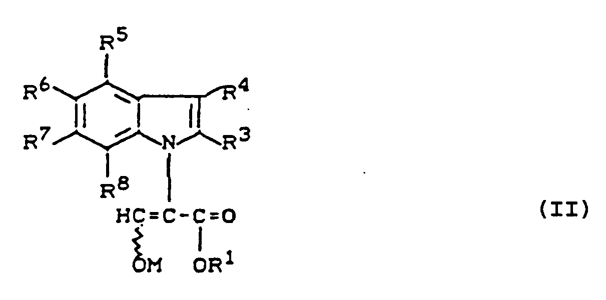 in which
M represents hydrogen, or represents an alkali metal cation, and
R¹, R³, R⁴, R⁵, R⁶, R⁷ and R⁸ have the abovementioned meaning
are reacted with alkylating agents of the formula (III)
R¹²-E¹ (III)
in which
R¹² represents alkyl or unsubstituted or substituted aralkyl and
E¹ represents an electron-attracting leaving group,
if appropriate in the presence of a diluent and if appropriate in the presence of a reaction auxiliary;
b) or in that, to obtain substituted 2-[6-(pyrimidinyl)-indol-1-yl]-acrylic esters of the general formula (Ib) 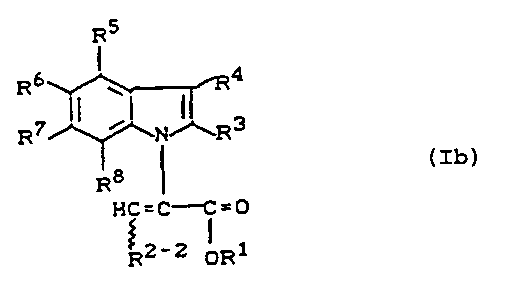 in which
R¹, R³, R⁴, R⁵, R⁶, R⁷ and R⁸ have the abovementioned meaning and
R²⁻² represents dialkylamino,
substituted acetic esters of the formula (IV) 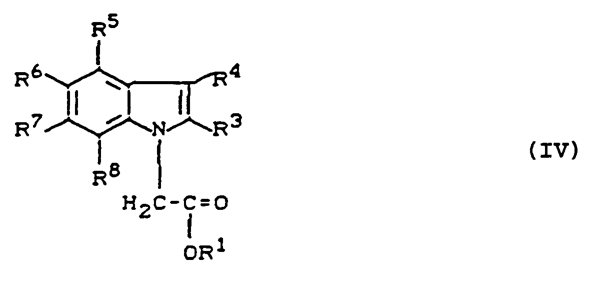 in which
R¹, R³, R⁴, R⁵, R⁶, R⁷ and R⁸ have the abovementioned meaning
are reacted with formamides of the formula (Va) 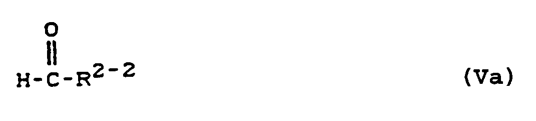 in which
R²⁻² has the abovementioned meaning,
or with formamide derivatives of the formula (Vb) 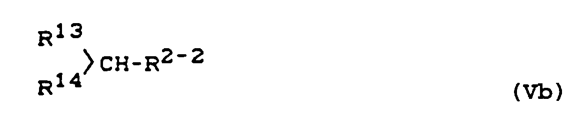 in which
R¹³ and R¹⁴ independently of one another represent alkoxy or dialkylamino and
R²⁻² has the abovementioned meaning,
if appropriate in the presence of a diluent;
c) or in that, to obtain substituted 2-[6-(pyrimidinyl)-indol-1-yl]-acrylic esters of the formula (Ic) 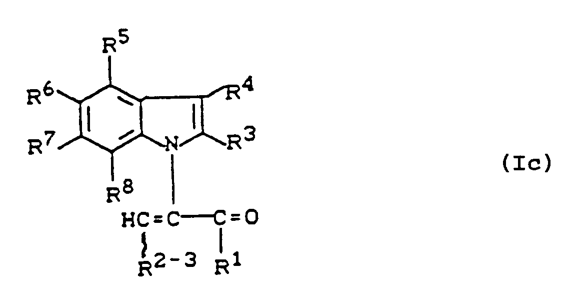 in which
R¹, R³, R⁴, R⁵, R⁶, R⁷ and R⁸ have the abovementioned meaning and
R²⁻³ represents alkylthio or unsubstituted or substituted aralkylthio,
ketocarboxylic acid derivatives of the formula (VI) 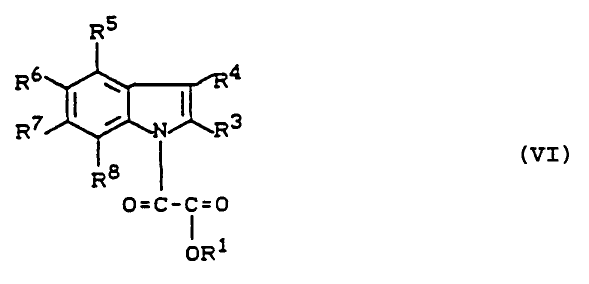 in which
R¹, R³, R⁴, R⁵, R⁶, R⁷ and R⁸ have the abovementioned meaning
are reacted with organometallic compounds of the formula (VII) 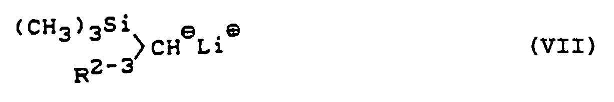 in which
R²⁻³ has the abovementioned meaning,
if appropriate in the presence of a diluent;
d) or in that, furthermore to obtain substituted 2-[6-(pyrimidinyl)-indol-1-yl]-acrylic esters of the formula (Ic) substituted acrylic esters of the formula (VIII) 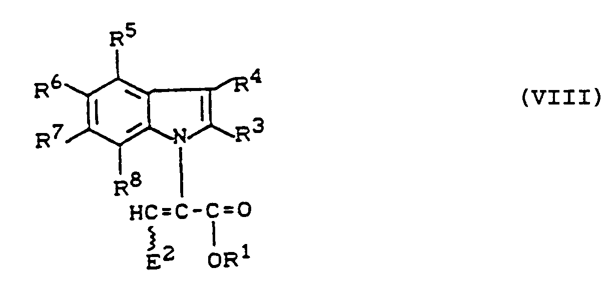 in which
R¹, R³, R⁴, R⁵, R⁶, R⁷ and R⁸ have the abovementioned meaning and
E² represents an electron-attracting leaving group,
are reacted with thiols of the formula (IX)
R²⁻³-H (IX)
in which
R²⁻³ has the abovementioned meaning,
if appropriate in the presence of a diluent and if appropriate in the presence of a reaction auxiliary.

4. Hydroxyacrylic ester derivatives of the general formula 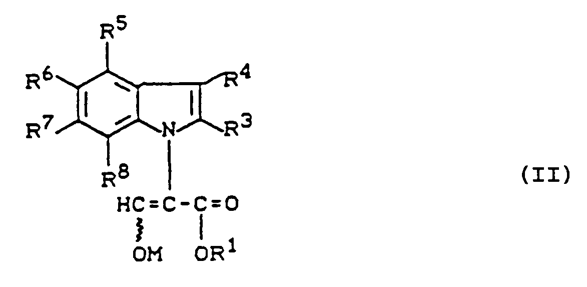 in which
M represents hydrogen, or represents an alkali metal cation, and
R¹, R³, R⁴, R⁵, R⁶, R⁷ and R⁸ have the meanings mentioned in Claim 1.

5. Process for the preparation of hydroxyacrylic ester derivatives of the general formula (II) 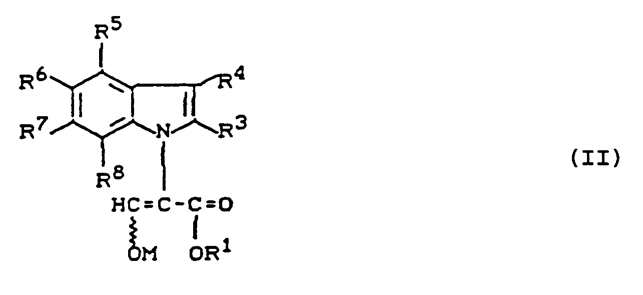 in which
M represents hydrogen, or represents an alkali metal cation, and
R¹, R³, R⁴, R⁵, R⁶, R⁷ and R⁸ have the meaning mentioned in Claim 1,
characterized in that substituted acetic ester derivatives of the formula (IV) 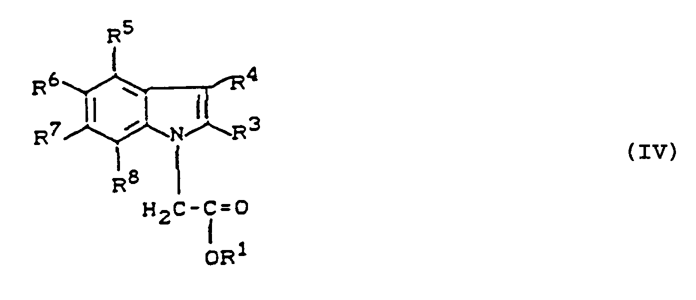 in which
R¹, R³, R⁴, R⁵, R⁶, R⁷ and R⁸ have the abovementioned meaning
are reacted with formic esters of the formula (X) 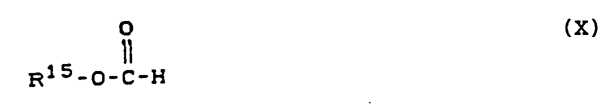 in which
R¹⁵ represents alkyl,
if appropriate in the presence of a diluent and if appropriate in the presence of a basic reaction auxiliary, at temperatures of from -20°C to +50°C.

6. Substituted acetic ester derivatives of the general formula (IV) 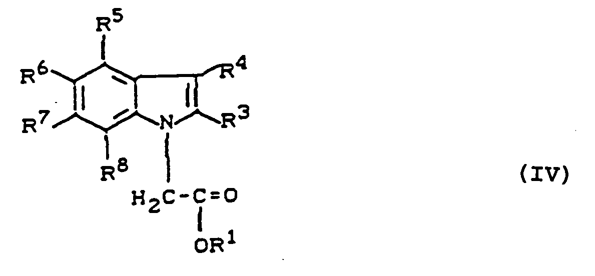 in which
R¹, R³, R⁴, R⁵, R⁶, R⁷ and R⁸ have the meaning mentioned in Claim 1.

7. Process for the preparation of acetic ester derivatives of the general formula (IV) 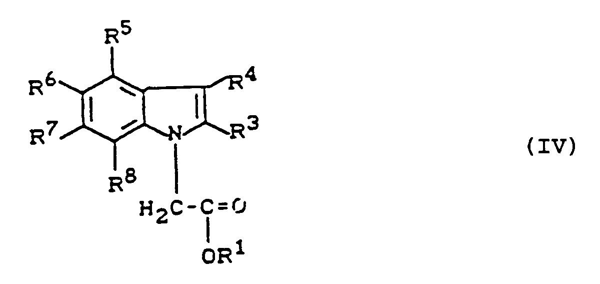 in which
R¹, R³, R⁴, R⁵, R⁶, R⁷ and R⁸ have the meaning mentioned in Claim 1,
characterized in that indole derivatives of the general formula (XI) 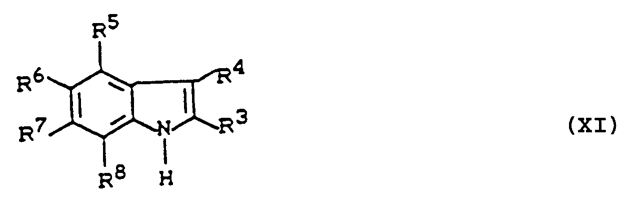 in which
R³, R⁴, R⁵, R⁶, R⁷ and R⁸ have the abovementioned meaning
are reacted with acetic acid derivatives of the general formula (XII)
E³-CH₂-COOR¹ (XII)
in which
R¹ has the abovementioned meaning and
E³ represents an electron-attracting leaving group,
if appropriate in the presence of a diluent and if appropriate in the presence of a basic auxiliary, at temperatures of between -20°C and +100°C.

8. Pesticides, characterized in that they contain at least one substituted 2-[6-(pyrimidinyl)-indol-1-yl]-acrylic ester of the formula (I) according to Claim 1.

9. Method of combating agricultural pests, characterized in that substituted 2-[6-(pyrimidinyl)-indol-1-yl]-acrylic esters of the formula (I) according to Claim 1 are allowed to act on agricultural pests and/or their habitat.

10. Use of substituted 2-[6-(pyrimidinyl)-indol-1-yl]-acrylic esters of the formula (I) according to Claim 1 for combating agricultural pests.

11. Process for the preparation of pesticides, characterized in that substituted 2-[6-(pyrimidinyl)-indol-1-yl]-acrylic esters of the formula (I) according to Claim 1 are mixed with extenders and/or surface-active agents.

12. Pesticides, characterized in that they contain at least one substituted 2-[6-(pyrimidinyl)-indol-1-yl]-acrylic ester of the formula (IV) according to Claim 6.

13. Method of combating agricultural pests, characterized in that substituted 2-[6-(pyrimidinyl)-indol-1-yl]-acrylic esters of the formula (IV) according to Claim 6 are allowed to act on agricultural pests and/or their habitat.

14. Use of substituted 2-[6-pyrimidinyl)-indol-1-yl)-acrylic esters of the formula (IV) according to Claim 6 for combating agricultural pests.

15. Process for the preparation of pesticides, characterized in that substituted 2-[6-(pyrimidinyl)-indol-1-yl]-acrylic esters of the formula (IV) according to Claim 6 are mixed with extenders and/or surface-active agents.

## Revendications

1. Esters d'acides 2-[6-(pyrimidinyl)-indole-1-yl]-acryliques substitués de formule générale (I) 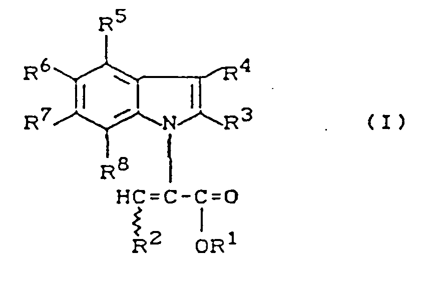 dans laquelle
R¹ est un groupe alkyle linéaire ou ramifié ayant 1 à 6 atomes de carbone ou un groupe benzyle,
R² est un groupe dialkylamino ayant 1 à 6 atomes de carbone dans chaque radical alkyle linéaire ou ramifié, un groupe alkoxy ou alkylthio linéaire ou ramifié ayant chacun 1 à 6 atomes de carbone ou un groupe benzyloxy ou benzylthio portant chacun le cas échéant un ou plusieurs substituants identiques ou différents, en considérant comme substituants du groupe phényle : un halogène, un groupe alkyle, alkoxy ou alkylthio linéaire ou ramifié ayant chacun 1 à 4 atomes de carbone, un groupe halogénalkyle, halogénalkoxy ou halogénalkylthio linéaire ou ramifié ayant chacun 1 à 4 atomes de carbone et 1 à 9 atomes d'halogènes identiques ou différents ou un groupe cycloalkyle ayant 3 à 7 atomes de carbone,
R³ et R⁴ représentent chacun, indépendamment l'un de l'autre, de l'hydrogène, un groupe cyano, du fluor, du chlore, du brome ou un groupe alkyle linéaire ou ramifié ayant 1 à 6 atomes de carbone,
R⁵, R⁶ et R⁸ représentent chacun, indépendamment les uns des autres, de l'hydrogène, du fluor, du chlore, du brome, un groupe cyano, nitro, un groupe alkyle, alkoxy ou alkylthio linéaire ou ramifié ayant chacun 1 à 4 atomes de carbone, un groupe halogénalkyle, halogénalkoxy ou halogénalkylthio linéaire ou ramifié ayant chacun 1 à 4 atomes de carbone et 1 à 9 atomes d'halogènes identiques ou différents, un groupe alkoxycarbonyle ou alkoximinoalkyle linéaire ou ramifié ayant chacun 1 à 4 atomes de carbone dans les parties alkyle individuelles, un groupe cycloalkyle ayant 3 à 7 atomes de carbone, un groupe phényle, benzyle, phénoxy, phénylthio, benzyloxy ou benzylthio non substitué ou portant chacun dans la partie aryle un à trois substituants, identiques ou différents, fluor, chlore, brome, alkyle, alkoxy alkylthio, halogénalkyle, halogénalkoxy ou halogénalkylthio ayant chacun 1 à 4 atomes de carbone et le cas échéant 1 à 9 atomes d'halogènes identiques ou différents, ou un groupe pyridyle, thiényle, furyle, pyridazinyle, pyrazinyle, thiazolyle, pyridylméthyle, thiénylméthyle, furylméthyle, pyridyloxy, thiényloxy, thiazolyloxy, pyridylméthyloxy, thiénylméthyloxy, thiénylthio, furylthio, pyridylméthylthio ou thiénylméthylthio portant chacun le cas échéant dans la partie hétéroaryle un ou deux substituants, identiques ou différents, halogéno, alkyle, alkoxy, alkylthio, halogénalkyle, halogénalkoxy ou halogénalkylthio ayant chacun 1 à 4 atomes de carbone et le cas échéant 1 à 9 atomes d'halogènes identiques ou différents, ou bien
R⁵ et R⁶ forment ensemble un groupe alcanediyle ayant 3 à 5 atomes de carbone ou un groupe alkylidènedioxy ayant 1 à 3 atomes de carbone et
R⁷ est l'un des groupements suivants 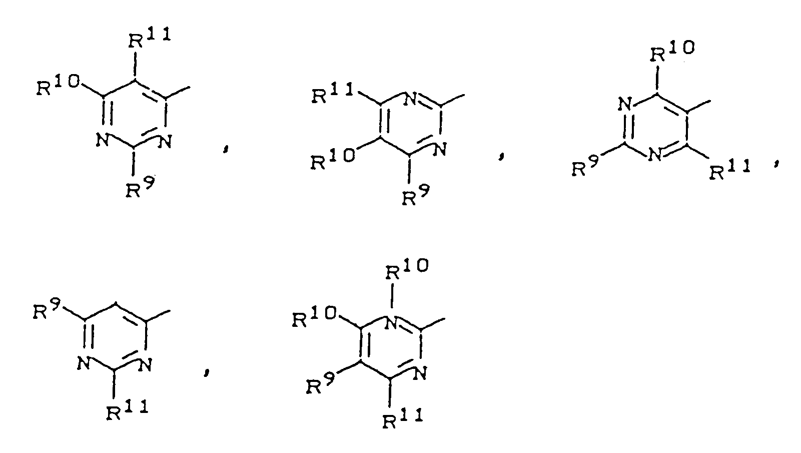 dans lesquels
R⁹, R¹⁰ et R¹¹ représentent chacun, indépendamment les uns des autres, de l'hydrogène, du fluor, du chlore, du brome, un groupe cyano, un groupe alkyle, alkoxy, alkylthio ou halogénalkyle linéaire ou ramifié ayant chacun 1 à 6 atomes de carbone et le cas échéant 1 à 13 atomes d'halogènes identiques ou différents, un groupe cycloalkyle ayant 3 à 7 atomes de carbone, un groupe alkoxycarbonyle linéaire ou ramifié ayant 1 à 6 atomes de carbone, un groupe dialkylaminocarbonyle, alkylaminocarbonyle, dialkylamino ou alkylamino ayant chacun 1 à 4 atomes de carbone, à chaîne droite ou ramifiée dans les parties alkyle individuelles et portant chacun le cas échéant des substituants identiques ou différents, ou un groupe phényle, benzyle, phényloxy, phénylthio, benzyloxy ou benzylthio non substitué chacun ou portant un à trois substituants identiques ou différents, ou un groupe pyrrolyle, thiényle, furyle, thiazolyle, isothiazolyle, oxazolyle, isoxazolyle, pyrazolyle, imidazolyle, pyridyle, pyrimidyle, pyridazinyle, pyrazinyle, triazinyle, pyrrolyloxy, thiényloxy, furyloxy, thiazolyloxy, isothiazolyloxy, oxazolyloxy, isoxazolyloxy, pyrazolyloxy, imidazolyloxy, pyridyloxy, pyrimidyloxy, pyridazinyloxy, pyrazinyloxy, pyrrolylthio, thiénylthio, furylthio, thiazolylthio, isothiazolylthio, oxazolylthio, isoxazolylthio, pyrazolylthio, imidazolylthio, pyridylthio, pyrimidylthio, pyridazinylthio ou pyrazinylthio non substitué ou portant un ou deux substituants identiques ou différents, en considérant dans chaque cas comme substituants : un halogène, un groupe alkyle, alkoxy, alkylthio, halogénalkyle, halogénalkoxy ou halogénalkylthio linéaire ou ramifié ayant chacun 1 à 4 atomes de carbone et chacun, le cas échéant, 1 à 9 atomes d'halogènes identiques ou différents, un groupe alkylcarbonylamino ayant 1 à 4 atomes de carbone dans la partie alkyle à chaîne droite ou ramifiée, un groupe dialkylamino ou dialkylaminocarbonyle ayant chacun 1 à 4 atomes de carbone dans chacune des parties alkyle linéaires ou ramifiées ainsi qu'un groupe phényle ou benzyle chacun éventuellement substitué, ou bien deux substituants voisins R⁹ et R¹⁰ ou R¹⁰ et R¹¹ ou R⁹ et R¹¹ forment ensemble un groupe alcanediyle ayant 3 ou 4 atomes de carbone.

2. Esters d'acides 2-[6-(pyrimidinyl)-indole-1-yl]-acryliques substitués de formule générale (I) suivant la revendication 1, dans laquelle
R¹ est un groupe alkyle linéaire ou ramifié ayant 1 à 4 atomes de carbone,
R² est un groupe dialkylamino ayant 1 à 4 atomes de carbone dans chacune des parties alkyle individuelle linéaires ou ramifiées, un groupe alkoxy ou alkylthio linéaire ou ramifié ayant 1 à 4 atomes de carbone ou un groupe benzyloxy ou benzylthio,
R³ et R⁴ sont identiques ou différents et représentent l'hydrogène, un groupe cyano, le fluor, le chlore, le brome, un groupe méthyle, éthyle, n-propyle, isopropyle, n-butyle, isobutyle, sec.-butyle ou tertiobutyle,
R⁵ et R⁶ sont identiques ou différents et représentent de l'hydrogène, du fluor, du chlore, du brome, un groupe cyano, nitro, méthyle, éthyle, n-propyle, isopropyle, n-butyle, isobutyle, sec.-butyle, tertiobutyle, méthoxy, éthoxy, n-propoxy, isopropoxy, méthylthio, trifluorométhyle, trifluorométhoxy, difluorométhoxy, trifluorométhylthio, méthoxycarbonyle, éthoxycarbonyle, méthoximinométhyle, éthoximinométhyle, méthoximinoéthyle, éthoximinoéthyle, cyclopentyle ou cyclohexyle, ou bien
R⁵ et R⁶ forment ensemble un groupement méthylènedioxy, 1,3-propandiyle ou 1,4-butanediyle,
R⁷ représente l'un des groupements suivants : 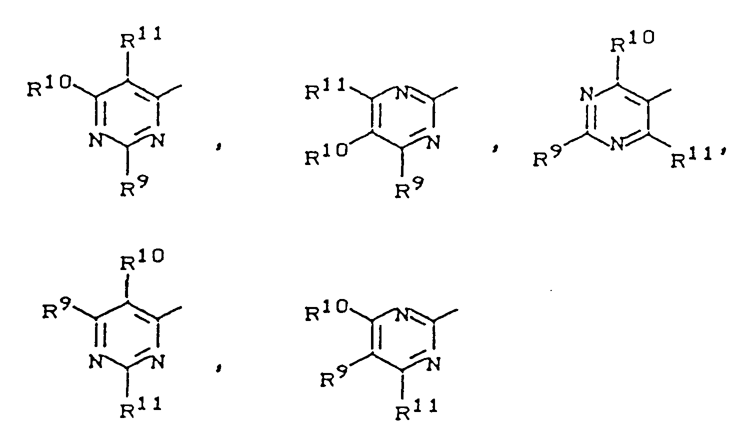 ou
R⁹ est de l'hydrogène, un groupe alkyle, alkoxy ou alkylthio linéaire ou ramifié ayant chacun 1 à 6 atomes de carbone, un groupe cyclopentyle, cyclohexyle, méthylamino, éthylamino, diméthylamino, diéthylamino, un groupe phényle, benzyle, phénoxy, phénylthio, benzyloxy ou benzylthio portant chacun le cas échéant un à trois substituants identiques ou différents ou un groupe pyrrolyle, thiényle, furyle, thiazolyle, isothiazolyle, oxazolyle, isoxazolyle, pyrazolyle, imidazolyle, triazinyle, pyridyle, pyrimidyle, pyridazinyle, pyrazinyle, pyridylméthyle, thiénylméthyle, furylméthyle, pyridyloxy, thiényloxy, thiazolyloxy, pyridylméthyloxy, thiénylméthyloxy, thiénylthio, furylthio, pyridylméthylthio ou thiénylméthylthio portant le cas échéant un ou deux substituants identiques ou différents, en considérant comme substituants, dans chaque cas, un halogène, un groupe méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylthio, éthylthio, n-propylthio, isopropylthio, n-butylthio, isobutylthio, sec.-butylthio, tertiobutylthio, méthylcarbonylamino, éthylcarbonylamino, méthylcarbonyle, éthylcarbonyle, n-propylcarbonyle, isopropylcarbonyle, n-butylcarbonyle, isobutylcarbonyle, sec.-butylcarbonyle, tertiobutylcarbonyle, halogénalkyle, halogénalkoxy ou halogénalkylthio ayant chacun 1 ou 2 atomes de carbone et 1 à 5 atomes de fluor ou de chlore, identiques ou différents, un groupe dialkylamino ou dialkylaminocarbonyle ayant 1 ou 2 atomes de carbone dans les parties alkyle individuelles, un groupe phényle ou benzyle non substitué ou portant un ou deux substituants identiques ou différents, en considérant comme substituants des groupes phényle et benzyle dans chaque cas le fluor, le chlore, un radical méthyle, méthoxy ou phénoxy, et
R¹⁰ et R¹¹ sont identiques ou différents et représentent de l'hydrogène, un groupe alkyle linéaire ou ramifié ayant 1 à 4 atomes de carbone, du chlore, du brome, un groupe cyano, méthoxycarbonyle, éthoxycarbonyle, diméthylaminocarbonyle, diéthylaminocarbonyle, méthylaminocarbonyle, éthylaminocarbonyle, méthoxy, éthoxy, méthylthio ou éthylthio, ou bien
R¹⁰ et R¹¹ forment ensemble un groupement méthylènedioxy, 1,3-propanediyle ou 1,4-butanediyle et
R⁸ représente de l'hydrogène, du fluor, du chlore, du brome, un groupe méthyle, éthyle, n-propyle, isopropyle, méthoxy, éthoxy, n-propoxy, isopropoxy, méthylthio, trifluorométhyle, trifluorométhoxy, difluorométhoxy, trifluorométhylthio, méthoxycarbonyle, éthoxycarbonyle, méthoximinométhyle ou éthoximinométhyle.

3. Procédé de production d'esters d'acides 2-[6-(pyrimidinyl)-indole-1-yl]-acryliques substitués de formule générale (I) 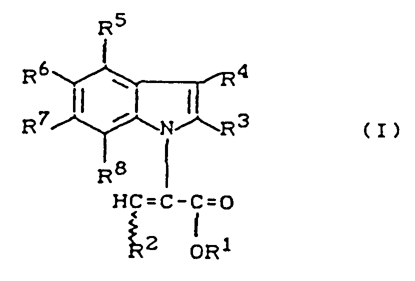 dans laquelle
R¹, R², R³, R⁴, R⁵, R⁶, R⁷ et R⁸ ont la définition indiquée dans la revendication 1,
caractérisé en ce que :
a) pour obtenir des esters d'acides 2-[6-(pyrimidinyl)-indole-1-yl]-acryliques substitués de formule générale (Ia) 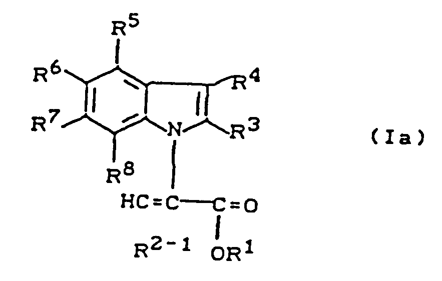 dans laquelle
R¹, R³, R⁴, R⁵, R⁶, R⁷ et R⁸ ont la définition indiquée ci-dessus et
R²⁻¹ représente un groupe alkoxy ou un groupe aralkyloxy non substitué ou substitué;
on fait réagir des esters d'acides hydroxyacryliques ou leurs sels de métaux alcalins de formule (II) 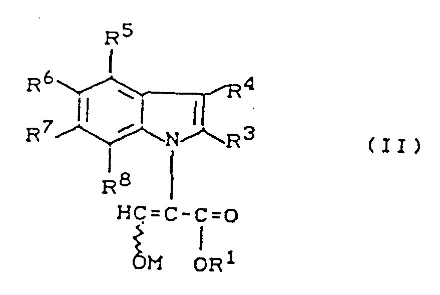 dans laquelle
M représente de l'hydrogène ou un cation de métal alcalin et
R¹, R³, R⁴, R⁵, R⁶, R⁷ et R⁸ ont la définition indiquée ci-dessus,
avec des agents alkylants de formule (III)
R¹²-E¹ (III)
dans laquelle
R¹² représente un groupe alkyle ou un groupe aralkyle non substitué ou substitué et
E¹ est un groupe partant attirant les électrons,
le cas échéant en présence d'un diluant et en la présence éventuelle d'une substance auxiliaire de réaction ;
ou en ce que
b) pour obtenir des esters d'acides 2-[6-(pyrimidinyl)-indole-1-yl]-acryliques substitués de formule générale (Ib) 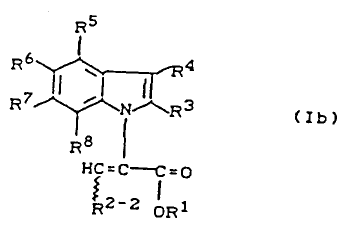 dans laquelle
R¹, R³, R⁴, R⁵, R⁶, R⁷ et R⁸ ont la définition indiquée ci-dessus et
R²⁻² représente un groupe dialkylamino,
on fait réagir des esters d'acide acétique substitués de formule (IV) 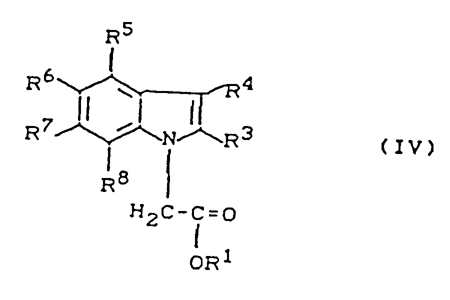 dans laquelle
R¹, R³, R⁴, R⁵, R⁶, R⁷ et R⁸ ont la définition indiquée ci-dessus,
avec des formamides de formule (Va) 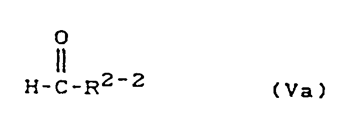 dans laquelle
R²⁻² a la définition indiquée ci-dessus,
ou avec des dérivés de formamide de formule (Vb) 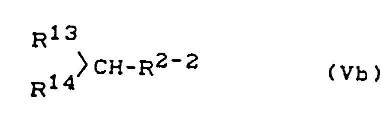 dans laquelle
R¹³ et R¹⁴ représentent, indépendamment l'un de l'autre, un groupe alkoxy ou dialkylamino et
R²⁻² a la définition indiquée ci-dessus,
le cas échéant en présence d'un diluant ; ou en ce que
c) pour obtenir des esters d'acides 2-[6-(pyrimidinyl)-indole-1-yl]-acryliques substitués de formule (Ic) 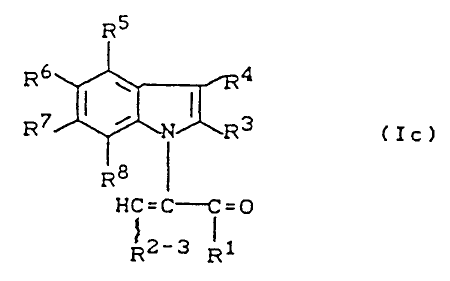 dans laquelle
R¹, R³, R⁴, R⁵, R⁶, R⁷ et R⁸ ont la définition indiquée ci-dessus, et
R²⁻³ est un groupe alkylthio ou un groupe aralkylthio non substitué ou substitué,
on fait réagir des dérivés d'acides cétocarboxyliques de formule (VI) 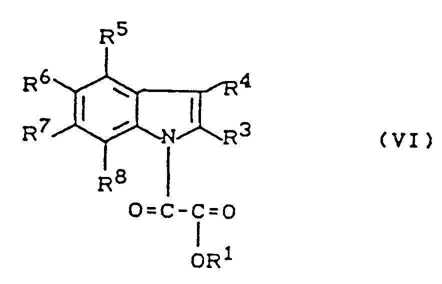 dans laquelle
R¹, R³, R⁴, R⁵, R⁶, R⁷ et R⁸ ont la définition indiquée ci-dessus,
avec des composés organométalliques de formule (VII) 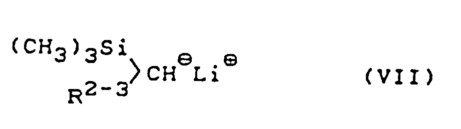 dans laquelle
R²⁻³ a la définition indiquée ci-dessus,
le cas échéant en présence d'un diluant ; ou en ce que
d) pour obtenir des esters d'acides 2-[6-(pyrimidinyl)-indole-1-yl]-acryliques substitués de formule (Ic),
on fait en outre réagir des esters d'acides acryliques substitués de formule (VIII) 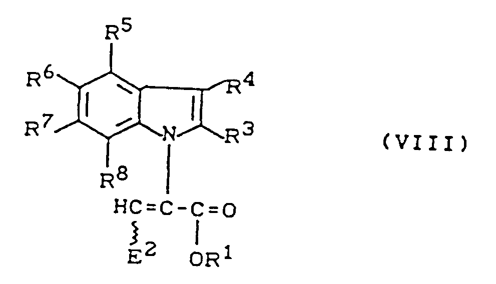 dans laquelle
R¹, R³, R⁴, R⁵, R⁶, R⁷ et R⁸ ont la définition indiquée ci-dessus et
E² est un groupe partant attirant les électrons,
avec des thiols de formule (IX)
R²⁻³-H (IX)
dans laquelle
R²⁻³ a la définition indiquée ci-dessus,
le cas échéant en présence d'un diluant et en la présence éventuelle d'une substance auxiliaire de réaction.

4. Dérivés d'esters d'acides hydroxyacryliques de formule générale 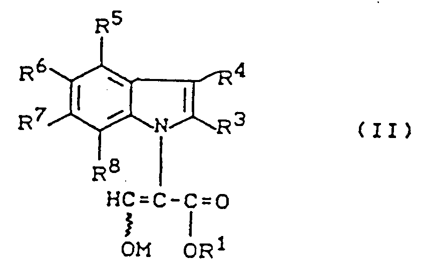 dans laquelle
M représente de l'hydrogène ou un ion de métal alcalin et
R¹, R³, R⁴, R⁵, R⁶, R⁷ et R⁸ ont la définition indiquée dans la revendication 1.

5. Procédé de production de dérivés d'esters d'acides hydroxyacryliques de formule générale (II) 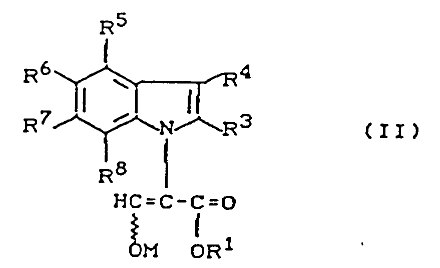 dans laquelle
M représente de l'hydrogène ou un cation de métal alcalin et
R¹, R³, R⁴, R⁵, R⁶, R⁷ et R⁸ ont la définition indiquée dans la revendication 1.
caractérisé en ce qu'on fait réagir des dérivés d'acide acétique substitués de formule (IV) 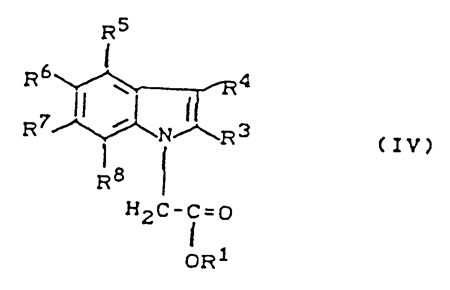 dans laquelle
R¹, R³, R⁴, R⁵, R⁶, R⁷ et R⁸ ont la définition indiquée ci-dessus,
avec des esters d'acide formique de formule (X) 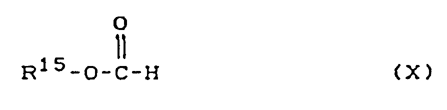 dans laquelle
R¹⁵ est un groupe alkyle,
le cas échéant en présence d'un diluant et en la présence éventuelle d'une substance auxiliaire basique de réaction, à des températures comprises entre -20°C et +50°C.

6. Dérivés d'esters d'acide acétique substitués de formule générale (IV) 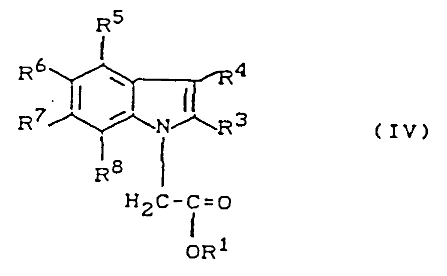 dans laquelle
R¹, R³, R⁴, R⁵, R⁶, R⁷ et R⁸ ont la définition indiquée dans la revendication 1.

7. Procédé de production de dérivés d'esters d'acide acétique de formule générale (IV) 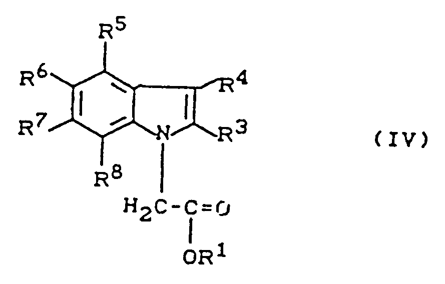 dans laquelle
R¹, R³, R⁴, R⁵, R⁶, R⁷ et R⁸ ont la définition indiquée dans la revendication 1,
caractérisé en ce qu'on fait réagir des dérivés d'indole de formule générale (XI) 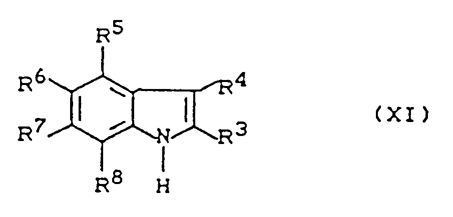 dans laquelle
R³, R⁴, R⁵, R⁶, R⁷ et R⁸ ont la définition indiquée ci-dessus, avec des dérivés d'acide acétique de formule générale (XII)
E³-CH₂-COOR¹ (XII)
dans laquelle
R¹ a la définition indiquée ci-dessus et
E³ est un groupe partant attirant les électrons,
le cas échéant en présence d'un diluant et en la présence éventuelle d'une substance auxiliaire basique à des températures comprises entre -20°C et +100°C.

8. Compositions pesticides, caractérisées par une teneur en au moins un ester d'acide 2-[6-(pyrimidinyl)-indole-1-yl]-acrylique substitué de formule (I) suivant la revendication 1.

9. Procédé pour combattre des parasites de l'agriculture, caractérisé en ce qu'on fait agir des esters d'acides 2-[6-(pyrimidinyl)-indole-1-yl]-acryliques substitués de formule (I) suivant la revendication 1 sur des parasites de l'agriculture et/ou sur leur milieu.

10. Utilisation d'esters d'acides 2-[6-(pyrimidinyl)-indole-1-yl]-acryliques substitués de formule (I) suivant la revendication 1 pour combattre des parasites de l'agriculture.

11. Procédé de préparation de compositions pesticides, caractérisé en ce qu'on mélange des esters d'acides 2-[6-(pyrimidinyl)-indole-1-yl]-acryliques substitués de formule (I) suivant la revendication 1 avec des diluants et/ou des agents tensio-actifs.

12. Compositions pesticides, caractérisées par une teneur en au moins un ester d'acide 2-[6-(pyrimidinyl)-indole-1-y1]-acrylique substitué de formule (IV) suivant la revendication 6.

13. Procédé pour combattre des parasites en agriculture, caractérisé en ce qu'on fait agir des esters d'acides 2-[6-(pyrimidinyl)-indole-1-yl]-acryliques substitués de formule (IV) suivant la revendication 6 sur des parasites de l'agriculture et/ou sur leur milieu.

14. Utilisation d'esters d'acides 2-[6-(pyrimidinyl)-indole-1-yl]-acryliques substitués de formule (IV) suivant la revendication 6 pour combattre des parasites de l'agriculture.

15. Procédé de préparation de compositions pesticides, caractérisé en ce qu'on mélange des esters d'acides 2-[6-(pyrimidinyl)-indole-1-yl]-acryliques substitués de formule (IV) suivant la revendication 6 avec des diluants et/ou des agents tensio-actifs.
